# EUROPÄISCHE PATENTSCHRIFT

(11) **EP 1 849 450 B1**
(45) Veröffentlichungstag und Bekanntmachung des Hinweises auf die Patenterteilung: **25.11.2009**
(21) Anmeldenummer: 06113260.1
(22) Anmeldetag: 28.04.2006
(51) Int. Cl.: A61K 6/083, A61K 6/00

(54) **Dentalwerkstoffe auf der Basis radikalisch polymerisierbarer Makromere mit antimikrobieller Wirkung**
Dental composition based on a radically polymerisable antibacterial macromonomer
Composition dentaire à base d'un macro-monomère antibactérien et polymérisable radicalairement

(43) Veröffentlichungstag der Anmeldung: 31.10.2007
(73) Patentinhaber: Ivoclar Vivadent AG, 9494 Schaan (LI)
(72) Erfinder: Rheinberger, Dr., Volker, FL-9490, Vaduz (LI); Salz, Dr., Ulrich, 88131, Lindau (DE); Zimmermann, Dr., Jörg, CH - 8046 Zürich (CH); Tiller, Dr., Jörg, D - 44225 Dortmund (DE); Waschinski, Dr., Christian, D - 69115 Heidelberg (DE); Poppe, Dr., Dirk, D - 60385 Frankfurt a.M. (DE)
(74) Vertreter: UEXKÜLL & STOLBERG

(56) Entgegenhaltungen:
- EP-A1- 0 980 682
- WASCHINSKI C.J.; TILLER J.C.: "Poly(oxazoline)s with Telechelic Antimicrobial Functions" BIOMACROMOLECULES, Bd. 6, 2005, Seiten 235-243, XP002405168
- WASCHINSKI, C.J.; HERDES, V.; SCHUELER, F.; TILLER, J.C.: "Influence of satellite groups on Telechic Antimicrobial Functions of Polyoxazolines" MACROMOLECULAR BIOSCIENCE, Bd. 5, 2005, Seiten 149-156, XP002405169
- FUCHS, A.D.; WASCHINSKI, C.J.; TILLER, J.C.: "Antimicrobial blockcopolymer emulsifier for contact-active polymer suspensions and surfaces" POLYMER PREPRINTS, Bd. 46, Nr. 2, 2005, Seite 1213, XP008070646

## Beschreibung

Die vorliegende Erfindung betrifft Dentalwerkstoffe auf der Basis antimikrobieller, radikalisch polymerisierbarer Makromere.

Die antimikrobielle Ausrüstung von Polymeren wird seit längerem durchgeführt, meist durch die rein physikalische Einmischung eines antimikrobiellen Wirkstoffs in eine entsprechende Werkstoffmatrix. So wird zum Beispiel in WO 98/48766 ein Zahnbeschichtungsmaterial zur Kariesprävention beschrieben, das als antimikrobiellen Wirkstoff Triclosan (2,4,4'-Trichlor-2'-hydroxydiphenylether) enthält. Auch in US 2003/0220416 werden Materialien für die zahnmedizinische Anwendung wie Prothesenkunststoffe und Befestigungszemente beschrieben, die antimikrobielle phenolische Substanzen wie Triclosan enthalten.

Die DE 198 13 686 offenbart ein Zahnwurzelfüllungsmaterial auf Guttaperchabasis, das als antimikrobiellen Wirkstoff Chlorhexidin freisetzt.

US 2005/0080158 beschreibt ein UV-härtendes Holzbeschichtungsmaterial, das Triclosan enthält, WO 2004/098658 schlägt u.a. die Verwendung von quaternären Ammoniumsalzen als antimikrobielle Wirkstoffe für polymere Beschichtungsmaterialien für den Baubereich vor.

Die rein physikalische Einmischung antimikrobieller Wirkstoffe in einen Werkstoff ist mit mehreren Problemen behaftet. Um seine antimikrobielle Wirkung zu erreichen, muß der Wirkstoff über die Funktionsdauer des Werkstoffs freigesetzt werden. In der Regel sind die Freisetzungsraten der Art, daß der Wirkstoff initial in sehr hoher Konzentration freigesetzt wird und danach der antimikrobielle Effekt weitgehend verloren geht. Nachteilig ist dabei zudem, daß bei hohen Wirkstoffkonzentrationen toxische Nebenwirkungen auftreten können, was besonders bei medizinischen Anwendungen wie z.B. Kontaktlinsen oder Dentalmaterialien unerwünscht ist. Des weiteren besteht bei kontinuierlicher oder erhöhter Wirkstoffreisetzung die Gefahr der Resistenzbildung. Aus diesem Grund ist man besonders für derartige Anwendungen dazu übergegangen, den Wirkstoff polymerisierbar zu machen um diesen bei der Aushärtung des Werkstoffs durch Homopolymerisation oder Copolymerisation mit einem anderen polymerisierbaren Monomeren im sich bildenden Polymeren zu immobilisieren.

US 5,536,861 und US 5,358,688 offenbaren Organosilikonmonomere für die Anwendung in Kontaktlinsen, das als antimikrobielle Gruppe eine quaternäre Ammoniumgruppe enthalten.

EP 0 663 409 offenbart Monomere für die Anwendung in Kontaktlinsen, die als wirksame Gruppe quaternäre Phosphoniumgruppen enthalten.

In EP 0 537 774 werden polymerisierbare Wirkstoffmonomere beschrieben, die eine quaternäre Ammoniumgruppe enthalten. Als polymerisierbare Gruppe werden (Meth)acrylfunktionalitäten verwendet, zwischen der polymerisierbaren Gruppe und der wirksamen Gruppe befindet sich ein Alkylenspacer mit 2 bis 18 C-Atomen.

In EP 0 705 590 und WO 01/90251 werden spezielle Zusammensetzungen für die dentale Anwendung beschrieben, die ein antimikrobielles, polymerisierbares Monomer gemäß EP 0 537 774 enthalten.

Nachteilig bei der Anwendung von polymerisierbaren Wirkstoffmonomeren ist, daß meist nur das Monomer eine antimikrobielle Wirkung aufweist, die nach der Polymerisation verloren geht. Häufig ist nur vorhandenes Restmonomer für die antimikrobielle Wirkung der Polymeren verantwortlich, so daß nach der Eluierung der nicht polymerisierten antimikrobiellen Monomere die antimikrobielle Wirkung der Werkstoffe verblaßt. Dadurch wird die antimikrobielle Langzeitwirkung deutlich reduziert, wodurch z.B. Arzneimittel oder Medizinprodukte ihre klinische Tauglichkeit zumindest teilweise verlieren.

Die DE 196 46 965 A1 offenbart Polymere mit antimikrobiellen Eigenschaften, die aus vinylisch polymerisierbaren Monomeren und aus Monomeren bestehen, bei denen mindestens ein langkettiger Alkylrest an eine quaternäre Ammoniumgruppe gebunden ist, die ihrerseits über einen hydrophilen Spacer mit einer Vinylfunktion verbunden ist. Die Polymere sollen sich zur Herstellung von Anstrichen wie z.B. Schiffsfarben und Formkörpern wie z.B. Sanitär- und Küchenoberflächen eignen.

Die WO 00/69926 offenbart ein Verfahren zur Herstellung inhärent antimikrobieller Polymere durch Polymerisation von Monomeren, die zumindest eine quartäre Aminofunktion besitzen. Die Monomere können einen Kohlenwasserstoffrest mit bis zu 50 Kohlenstoffatomen aufweisen und haben eine Molmasse unter 900. Die Polymere sollen sich zur Herstellung von Lacken, Schutzanstrichen und Beschichtungen z.B. für Schiffsrümpfe, Toilettenartikel, Kontaktlinsen, Membranen und Implantate eignen.

Der Erfindung liegt die Aufgabe zugrunde, Dentalmaterialien bereitzustellen, die im polymerisierten Zustand eine hohe antimikrobielle Wirkung haben und keine antimikrobiell wirksame Komponente freisetzen.

Diese Aufgabe wird erfindungsgemäß durch Dentalwerkstoffe gelöst, die mindestens eine Verbindung der Formel (I) enthalten,

[PG]ₘ-R¹-Z-SP-Y-R²-[WG]ₚ (I)

in der die Variablen die folgenden Bedeutungen haben:
- m =: 1, 2 oder 3;
- p ₌: 1, 2 oder 3;
- R¹ =: entfällt, ein linearer oder verzweigter C₁ bis C₂₀-Alkylenrest, der ein- oder mehrfach durch O, S, NH, SiR'₂, CONH, CONR', COO und/oder OCONH unterbrochen sein kann, ein substituierter oder unsubstituierter, aromatischer C₆ bis C₁₄-Rest oder eine Kombination davon;
- R² =: entfällt, ein linearer oder verzweigter C₁ bis C₂₀-Alkylenrest, der ein- oder mehrfach durch O, S, NH, SiR'₂, CONH, CONR', COO und/oder OCONH unterbrochen sein kann, ein substituierter oder unsubstituierter, aromatischer C₆ bis C₁₄-Rest oder eine Kombination davon;
- PG =: eine radikalisch polymerisierbare Gruppe;
- SP =: ein polymerer Spacer, der vorzugsweise aus Polyethylenglycol-, Polypropylenglycol-, insbesondere Polyglycerin-, Polyalkyloxazolinen-, Polyethyleniminen-, Polyacrylsäure-, Polymethacrylsäure-, Polyvinylalkohol-, Polyvinylacetat-, Poly-(2-hydroxyethyl)acrylat-, Poly-(2-hydroxyethyl)methacrylatgruppen, hydrophilen Polypeptidgruppen und Copolymeren, insbesondere Blockcopolymeren der entsprechenden Monomere ausgewählt ist;
- WG =: eine antimikrobiell wirksame Gruppe;
- Z =: entfällt, O, S, eine Ester-, Amid- oder Urethangruppe;
- Y =: entfällt, O, S, eine Ester-, Amid- oder Urethangruppe.

R' steht jeweils unabhängig für einen linearen oder verzweigten C₁ bis C₂₀-Alkylrest, einen substituierten oder unsubstituierten Phenyl- oder Benzylrest.

Die bei R¹, R², und R' gegebenenfalls vorhandenen Substituenten sind unabhängig voneinander vorzugsweise aus C₁-C₆-Alkyl, C₁-C₆-Alkoxyl, Aryl, Benzyl, F-, C1-, Br₋, I-, HO-, HOOC-, C₁-C₆-Alkyl-OOC-, H₂NOC-, HR"OC- und R"₂OC- ausgewählt, wobei R" ein C₁-C₂₀-Alkylen-, Phenyl- oder Benzylrest ist.

Durch die Formel (I) werden nur solche Verbindungen erfaßt, die mit der chemischen Valenzlehre vereinbar sind. Der Hinweis, daß eine Rest z.B. durch O, S, NH, SiR', CONH, CONR', COO, OCONH etc. unterbrochen sein kann, ist so zu verstehen, daß diese Atome oder Gruppen in die Kohlenstoffkette des Restes eingeschoben werden, d.h. beidseitig von Kohlenstoffatomen begrenzt werden. Die Anzahl dieser Fremdatome oder Gruppen ist daher um mindestens 1 kleiner als die Zahl der Kohlenstoffatome, und die Fremdatome oder Gruppen können nicht endständig sein.

Unter Kombinationen von Alkylenresten und aromatischen Gruppen werden vorzugsweise Alkylen-Arylen-, Alkylen-Arylen-Alkylen- und Arylen-Alkylen-Arylen-Gruppen, insbesondere -CH₂-Ph- und -CH₂-Ph-CH-Gruppen verstanden.

Die Verbindung der Formel (I) hat ein Molekulargewicht von mindestens 1.000 g/mol, vorzugsweise mindestens 2.500 g/mol und besonders bevorzugt mindestens 5.000 g/mol. Die Verbindung der Formel (I) enthält mindestens eine radikalisch polymerisierbare Gruppe und kann daher durch radikalische Polymerisation gehärtet werden. Sie wird im folgenden auch als Makromer bezeichnet.

Bevorzugte polymerisationsfähige Gruppen (PG) sind Vinyl-CH₂=CH-, Allyl- CH₂=CH-CH₂-, Vinylether- CH₂=CH-O-, Styryl-CH₂=CH-(Ph)-, Vinylcyclopropylgruppen und/oder Gruppen der Formel (II), 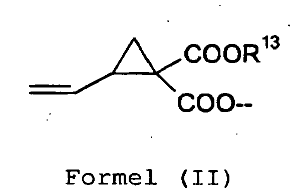 in der R¹³ Methyl oder Phenyl ist.

Ganz besonders bevorzugte polymerisierbare Gruppe PG sind (Meth)acryloyl CH₂=C(CH₃)-COO- und (Meth)acrylamidgruppen CH₂=C(CH₃)-CO-NH-.

Die antimikrobielle Gruppe (WG) kann eine primäre, sekundäre, oder tertiäre Aminogruppe, eine kationische primäre, sekundäre, tertiäre oder quartäre Ammonium-, Phosphonium- oder Sulfoniumgruppe, eine Biguanidingruppe, ein antimikrobielles Peptid, ein Phenol- oder Polyphenolrest und/oder ein Antibiotikum sein. Bevorzugte antimikrobielle Gruppen sind quartäre Ammoniumgruppen, Phosphoniumgruppen und Sulfoniumgrupppen.

Unter primären, sekundären, tertiären und quartären Ammoniumgruppen werden Gruppen der Formel (-N⁺RH₃) (-N⁺R₂H₂), (-N⁺R₃H) und (-N⁺R₄) verstanden.

Bevorzugte Antibiotika sind Penicillingruppen, insbesondere:

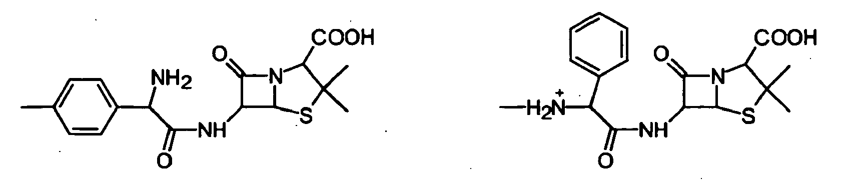

Die antimikrobielle Gruppe WG kann eine positive Ladung aufweisen. Diese wird durch Anionen A⁻ ausgeglichen. Bevorzugte Anionen A⁻ sind Cl⁻, Br⁻, I⁻, Triflat (Trf), Mesylat (Mes) und Tosylat (Tos):

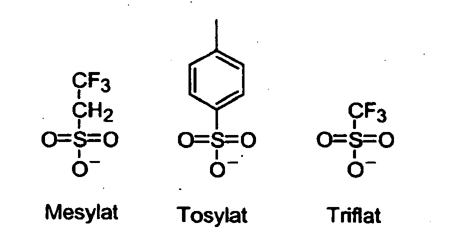

Gemäß einer bevorzugten Ausführungsform ist der Spacer SP eine Gruppe mit der Formel -[R³]_{q}-, in der R³ -[CH₂-CH₂-O]-, -[CH(CH₃)-CH₂-O]-, vorzugsweise -[CH(OH)CH₂]-, -[CH(COOH)CH₂] -[CH(COOCH₃)CH₂]-, -[C(CH₃) (COOH)CH₂]-, -[C(CH₃) (COOCH₃)CH₂]-, -[CH(COOCH₂CH₂OH)CH₂]-, - [C(CH₃) (COOCH₂CH₂OH)CH₂]-, -[CH(OCOCH₃)CH₂]-, -[CH₂-CH(OH)-CH₂-O]- und insbesondere - [R⁴-N(-CO-R⁶)-R⁵] - oder eine Kombination daraus ist, wobei
- R⁴ =: entfällt, ein linearer oder verzweigter C₁ bis C₂₀-Alkylenrest, der durch ein oder mehrere O-Atome unterbrochen sein kann, ein substituierter oder unsubstituierter, aromatischer C₆ bis C₁₄-Rest, vorzugsweise - (CH₂)₁₋₄- und insbesondere entfällt;
- R⁵ =: entfällt, ein linearer oder verzweigter C₁ bis C₂₀-Alkylenrest, der durch ein oder mehrere O-Atome unterbrochen sein kann, ein substituierter oder unsubstituierter, aromatischer C₆ bis C₁₄-Rest, vorzugsweise - (CH₂)₁₋₄- oder entfällt, insbesondere -(CH₂)₂-;
- R⁶ =: ein linearer oder verzweigter C₁ bis C₂₀-Alkylrest, ein substituierter oder unsubstituierter, aromatischer C₆ bis C₁₄-Rest, vorzugsweise C₁-C₃-Alkyl, insbesondere Methyl;
- q =: 10 bis 15.000, vorzugsweise 10 bis 10.000, besonders bevorzugt 10 bis 5.000, ganz besonders bevorzugt 15 bis 2.000.

R⁴ und R⁵ entfallen vorzugsweise nicht gleichzeitig. Eine ganz besonders bevorzugte Gruppe der Formel -[R³]- ist -[N(COCH₃)-CH₂-CH₂]-.

Die bei R⁴, R⁵, und R⁶ gegebenenfalls vorhandenen Substituenten sind unabhängig voneinander vorzugsweise aus C₁-C₆-Alkyl, C₁-C₆-Alkoxyl, Aryl, Benzyl, F-, Cl-, Br-, I-, HO-, HOOC-, C₁-C₆-Alkyl-OOC-, H₂NOC-, HR"OC- und R"₂OC- ausgewählt, wobei R" ein C₁-C₂₀-Alkylen-, Phenyl- oder Benzylrest ist.

Verbindungen, bei denen der Spacer eine Gruppe der Formel - [R³]_{q}- ist, lassen sich durch die allgemeine Formel (I') darstellen:

[PG]ₘ-R¹-Z-[R³]_{q}-Y-R²-[WG]ₚ Formel (I')

in der die übrigen Variablen die oben angegebenen Bedeutungen haben.

Bevorzugte Definitionen der übrigen Variablen, die unabhängig voneinander gewählt werden können, sind:
- m =: 1 oder 2, insbesondere 1;
- p =: 1 oder 2, insbesondere 1;
- R¹ =: entfällt, ein linearer oder verzweigter C₁ bis C₄-Alkylenrest, der einfach durch COO unterbrochen sein kann, Phenylen, Benzylen, -CH₂-Ph-CH₂-;
- R² =: entfällt, ein linearer oder verzweigter C₁ bis C₄-Alkylenrest, der einfach durch COO unterbrochen sein kann, Phenylen, Benzylen, -CH₂-Ph-CH₂-;
- PG =: CH₂=CR¹⁰-CO-X- mit R¹⁰ = H oder CH₃ und X = O, NH, NR¹², wobei R¹² ein linearer C₁ bis C₅-Alkylrest, vorzugsweise Methyl ist, CH₂=CH- oder Vinylcyclopropyl;
- Z =: entfällt, O, S, COO, vorzugsweise entfällt, O, S;
- Y =: entfällt, O, S, COO; vorzugsweise entfällt;
- A⁻ =: Cl⁻, Br⁻, I⁻, Mesylat (Mes), Tosylat (Tos) oder Triflat (Trf);
WG =-N⁺R⁷R⁸R⁹ A⁻, mit
- R⁷ =: H, ein linearer oder verzweigter C₁ bis C₂₀-Alkylrest, oder R⁷ bildet zusammen mit R⁸ und dem Stickstoffatom an den es gebunden ist ein aromatisches oder heteroaromatisches Ringsystem oder einen aromatischen oder nicht aromatischen Ring, vorzugsweise ein C₁ bis C₆-Alkylrest, besonders bevorzugt CH₃; 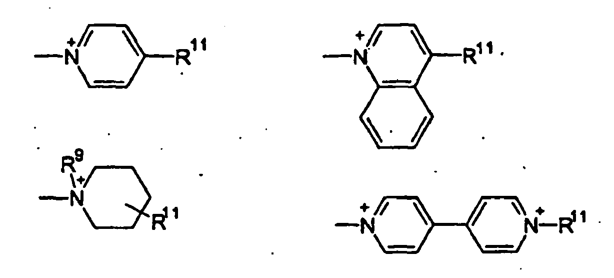 wobei R¹¹ ein linearer oder verzweigter C₁ bis C₂₀-Alkylrest ist;
- R⁸ =: H, ein linearer oder verzweigter C₁ bis C₂₀-Alkylrest oder R⁸ bildet zusammen mit R⁷ und dem Stickstoffatom an den es gebunden ist ein aromatisches oder heteroaromatisches Ringsystem oder einen aromatisches oder nicht aromatischen Ring, vorzugsweise ein C₁ bis C₆-Alkylrest, besonders bevorzugt CH₃;
- R⁹ =: entfällt, H, ein linearer oder verzweigter C₁ bis C₃₁-alkylrest oder ein linearer oder verzweigter C₁ bis C₂₀-Alkylrest, vorzugsweise -(CH₂)-ᵣ-H;
- r =: 5 bis 30, vorzugsweise 10 bis 30, besonders bevorzugt 10 bis 20;
-P⁺R^{7'}R^{8'}R^{9'} A⁻, mit
- R^{7'} =: H, ein linearer oder verzweigter C₁ bis C₂₀-Alkylrest, vorzugsweise ein C₁ bis C₆-Alkylrest, besonders bevorzugt CH₃;
- R^{8'} =: H, ein linearer oder verzweigter C₁ bis C₂₀-Alkylrest, vorzugseise Methyl;
- R^{9'} =: entfällt, H, ein linearer oder verzweigter C₁ bis C₂₀-Alkylrest, oder -(CH₂)ᵣ-H;
- r =: 5 bis 30, vorzugsweise 10 bis 30, besonders bevorzugt 10 bis 20; oder 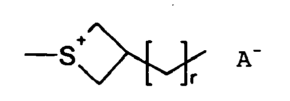
- r =: 5 bis 30, vorzugsweise 10 bis 30, besonders bevorzugt 10 bis 20.

Besonders bevorzugt sind naturgemäß solche Verbindungen, bei denen alle Variablen eine der bevorzugten und insbesondere der besonders bevorzugten Bedeutungen haben.

Besonders bevorzugte Verbindungen der Formel (I') sind: 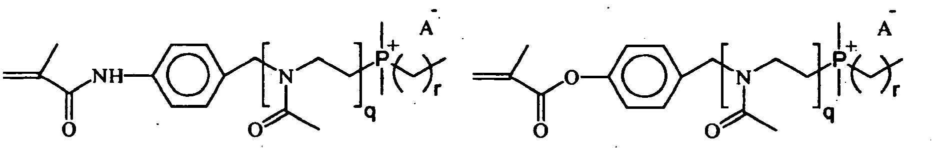 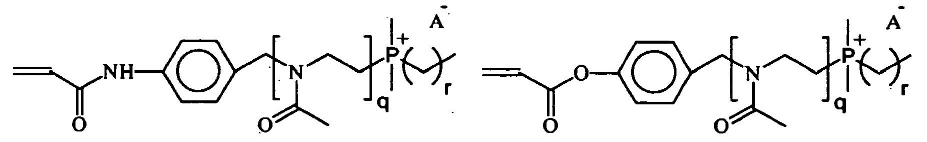 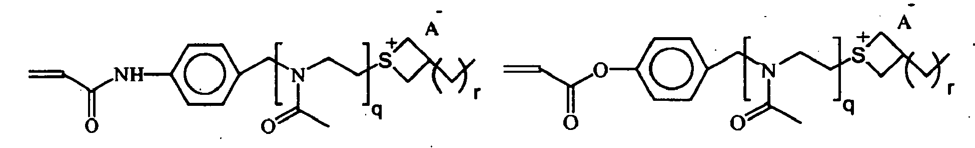 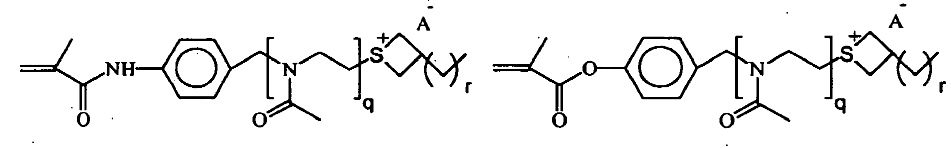
- A⁻ =: Cl⁻, Br⁻, I⁻, Trf, Mes, Tos
- q =: 10 bis 10.000
- r =: 10 bis 20

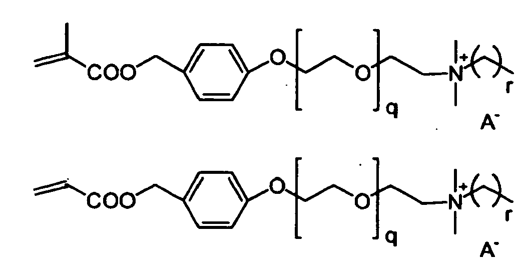

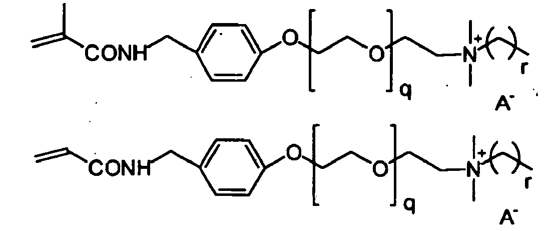

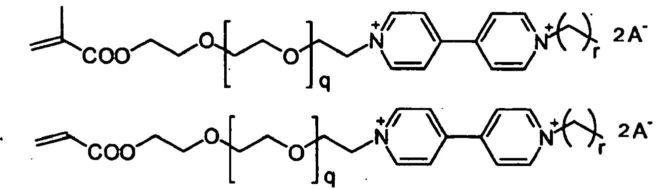

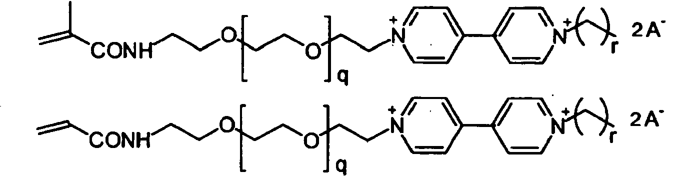

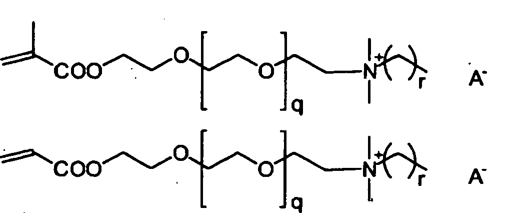

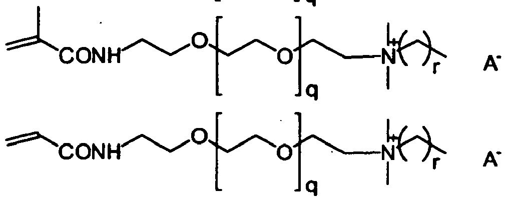

- A⁻ =: Cl⁻, Br⁻, I⁻, Trf, Mes, Tos
- q =: 45 bis 15.000
- r =: 10 bis 20

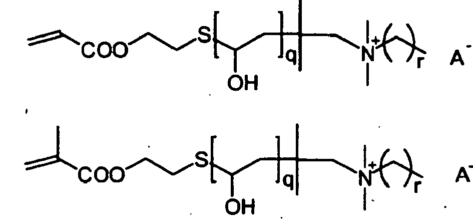

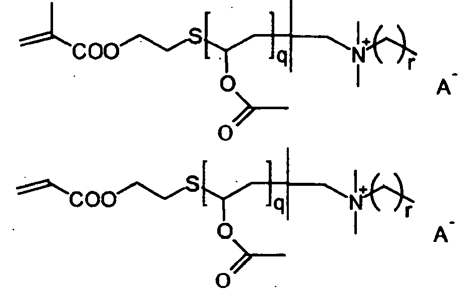

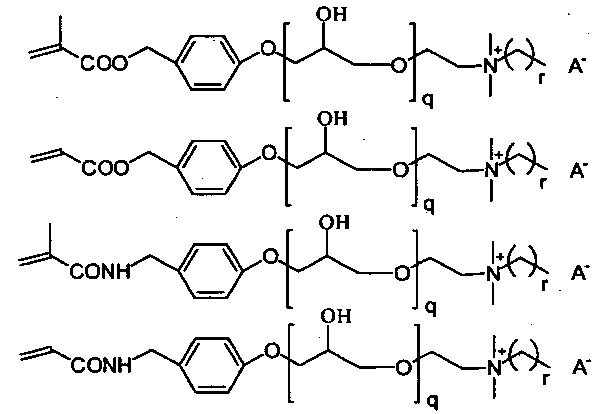

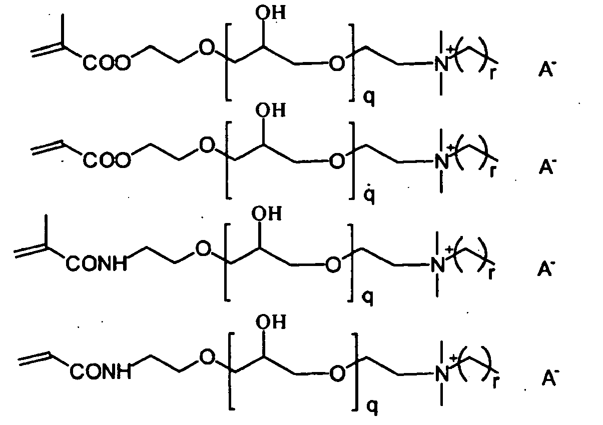

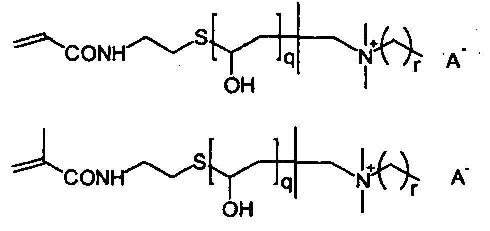

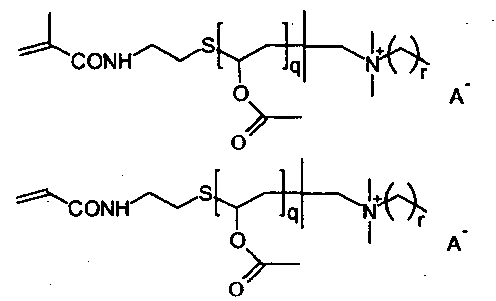

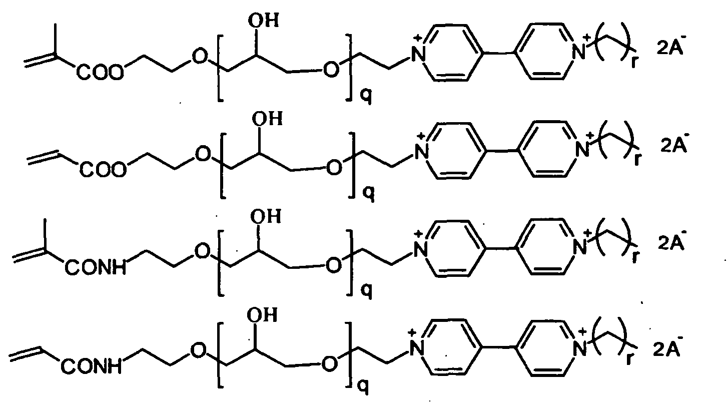

- A⁻ =: Cl⁻, Br⁻, I⁻, Trf, Mes, Tos
- q =: 10 bis 10.000
- r =: 10 bis 20

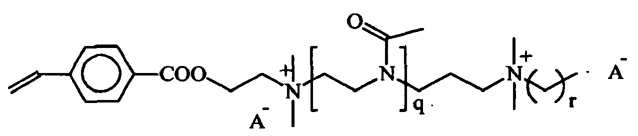

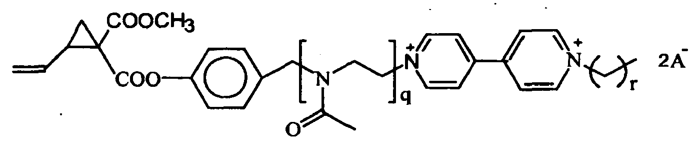

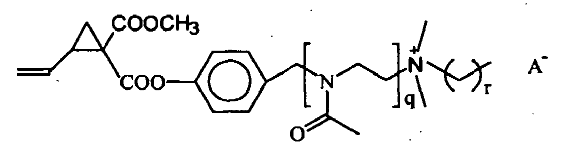

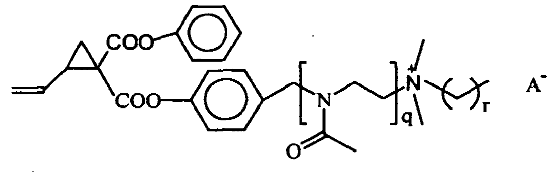

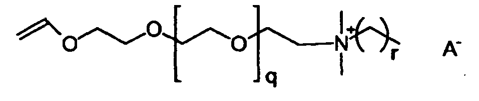

- A⁻ =: Cl⁻, Br⁻, I⁻, Trf, Mes, Tos
- q =: 10 bis 10.000
- r =: 10 bis 20

Ganz besonders bevorzugt sind Verbindungen der Formel (I") 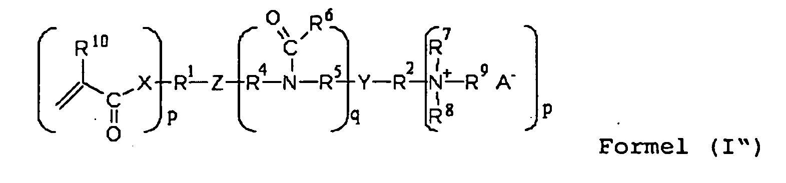 in der die Variablen die oben angegebenen Bedeutungen und bevorzugten Bedeutungen haben.

Bevorzugte Beispiele der Makromere der Formel (I") mit einem Polyoxazolinspacer sind: 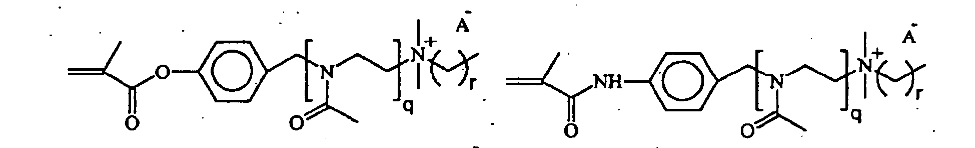 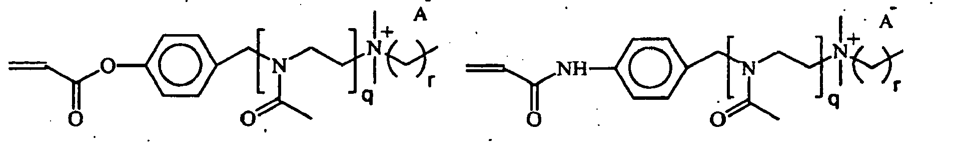 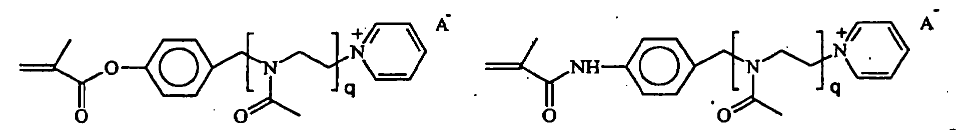 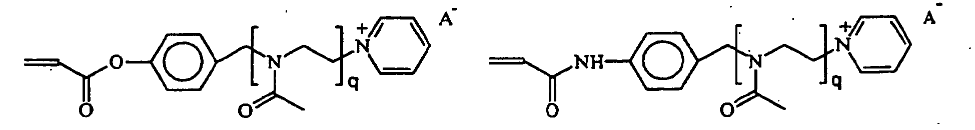 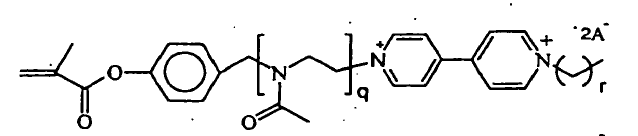 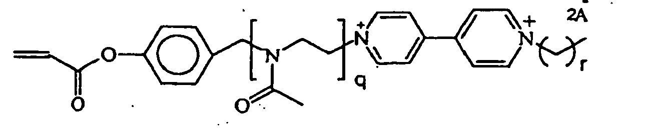 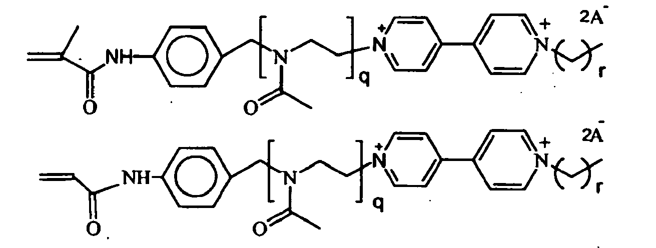 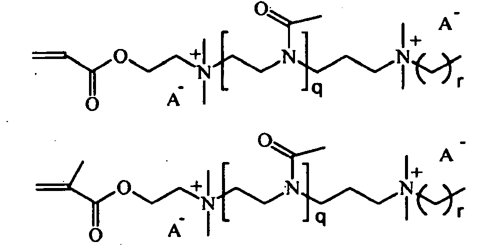 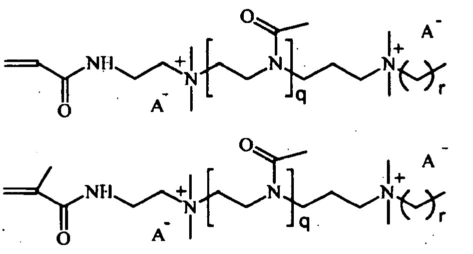
- A⁻ =: Cl⁻, Br⁻, I⁻, Trf, Mes, Tos
- q =: 10 bis 10.000
- r =: 10 bis 20

Die erfindungsgemässen antimikrobiellen, polymerisierbaren Makromere der allgemeinen Formel (I) lassen sich durch bekannte mehrstufige Syntheseverfahren herstellen. Beispielsweise lassen sich die besonders bevorzugten antimikrobiellen, polymerisierbaren Makromere der Formel (I") mit einem Polyoxazolinspacer auf die folgenden Weisen herstellen.

Bei einer ersten Synthesevariante wird in der ersten Stufe der polymere Spacer (SP) durch Polymerisation von 2-Alkyl-1,3-oxazolin aufgebaut. Die Steuerung des Polymerisationsgrades und somit der Spacerlänge erfolgt über das Monomer-Initiator-Verhältnis [M₀]/[I] und die Reaktionszeit. Für den Polymerisationsabbruch wird ein tertiäres Amin verwendet, wobei sich daraus die entsprechende, antimikrobiell wirksame quaternäre Ammoniumgruppe (WG) bildet. Nach Entfernung der Schutzgruppe wird durch Umsetzung des freiwerdenden Amins mit Methacrylsäure- oder Acrylsäurechlorid die polymerisationsfähige Gruppe (PG) eingeführt. Es bildet sich das entsprechende Methacryl- bzw. Acrylamid.

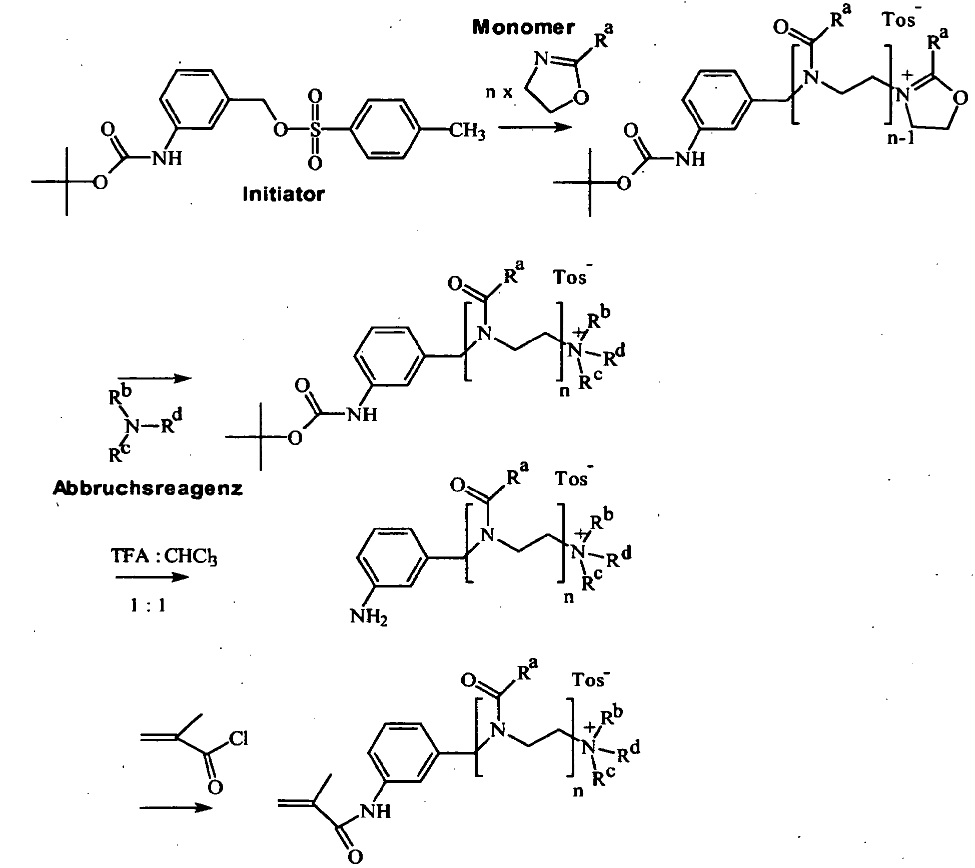

Bei einer zweiten Synthesevariante wird der polymere Spacer ebenfalls durch Polymerisation von 2-Alkyl-1,3-oxazolinen aufgebaut. Auch in diesem Fall wird wie bei der ersten Synthesevariante der Polymerisationsgrad und somit die Spacerlänge über das Monomer-Initiator-Verhältnis und die Reaktionszeit kontrolliert. Die antimikrobiell wirksame quartäre Ammoniumgruppe (WG) wird durch den Initiator eingeführt, zu der polymerisationsfähigen Gruppe (PG) am anderen Polymerende gelangt man durch Terminierung der Reaktion mit Aminen, die polymerisationsfähige Gruppen enthalten.

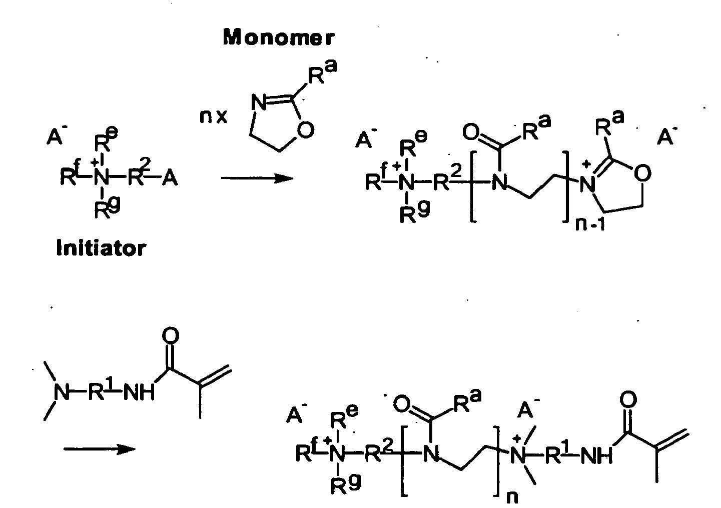

Die Makromere der Formel (I) können durch Homopolymerisation oder durch Copolymerisation mit weiteren Makromeren der Formel (I) und/oder anderen radikalisch polymerisierbaren Monomeren in Polymere überführt werden, in denen die antimikrobielle Komponente immobilisiert ist und nicht ausgewaschen wird.

Die erfindungsgemäßen Dentalwerkstoffe können neben den Makromeren der Formel (I) weitere radikalisch polymerisierbare Monomere mit einer oder mehreren radikalisch polymerisierbaren Gruppen enthalten. Dentalwerkstoffe, die mindestens ein weiteres radikalisch polymerisierbares Monomer mit 2 oder mehr, vorzugsweise 2 bis 3 radikalisch polymerisierbaren Gruppen enthalten, sind besonders bevorzugt.

Bevorzugte zusätzliche Monomere sind mono- oder mehrfach funktionelle (Meth)acrylate oder (Meth)acrylamide ((Meth)acrylverbindungen). Unter monofunktionellen (Meth)acrylverbindungen werden Verbindungen mit einer, unter mehrfach funktionellen (Meth)acrylverbindungen Verbindungen mit zwei oder mehr, vorzugsweise 2 bis 3 (Meth)acrylgruppen verstanden. Mehrfach funktionelle Monomere haben vernetzende Eigenschaften.

Bevorzugte monofunktionelle (Meth)acrylverbindungen sind kommerziell verfügbare monofunktionelle Monomere, wie Methyl-, Ethyl-, Butyl-, Benzyl-, Furfuryl- oder Phenyl(meth)acrylat sowie 2-Hydroxyethyl- oder -propyl(meth)acrylat.

Besonders bevorzugt sind hydrolysestabile Monomere wie hydrolysestabile Mono(meth)acrylate, z.B. Mesitylmethacrylat oder 2-(Alkoyxymethyl)acrylsäuren, z.B. 2-(Ethoxymethyl)acrylsäure, 2-(Hydroxymethyl)acrylsäure, N-mono- oder -disubstitiuierte Acrylamide, wie z.B. N-Ethylacrylamid, N,N-Dimethacrylamid, N-(2-Hydroxyethyl)acrylamid oder N-Methyl-N-(2-hydroxyethyl)acrylamid, und N-monosubstituierte Methacrylamide, wie z.B. N-Ethylmethacrylamid oder N-(2-Hydroxyethyl)methacrylamid sowie außerdem N-Vinylpyrrolidon und Allylether. Diese Monomere sind bei Raumtemperatur flüssig und eignen sich daher auch als Verdünner.

Bevorzugte mehrfach funktionelle Monomere sind Bisphenol-A-di(meth)acrylat, Bis-GMA (ein Additionsprodukt aus Methacrylsäure und Bisphenol-A-diglycidylether), ethoxyliertes Bisphenol-A-di(meth)acrylat, UDMA (ein Additionsprodukt aus 2-Hydroxyethylmethacrylat und 2,2,4-Trimethylhexamethylendiisocyanat), Di-, Tri- oder Tetraethylenglycoldi(meth)acrylat, Trimethylolpropantri(meth)acrylat, Pentaerythrittetra(meth)acrylat, sowie Butandioldi(meth)acrylat, 1,10-Decandioldi(meth)acrylat oder 1,12-Dodecandioldi(meth)acrylat.

Besonders bevorzugt sind weiterhin hydrolysestabile Vernetzermonomere, wie z.B. vernetzende Pyrrolidone, wie z.B. 1,6-Bis-(3-vinyl-2-pyrrolidonyl)-hexan, oder kommerziell zugängliche Bisacrylamide wie Methylen- oder Ethylenbisacrylamid, Bis-(meth)acrylamide, wie z.B. N,N'-Diethyl-1,3-bis-(acrylamido)-propan, 1,3-Bis-(methacrylamido)-propan, 1,4-Bis-(acrylamido)-butan oder 1,4-Bis-(acryloyl)-piperazin, die durch Umsetzung aus den entsprechenden Diaminen mit (Meth)acrylsäurechorid synthetisiert werden können.

Die erfindungsgemäßen Dentalwerkstoffe enthalten vorzugsweise auch mindestens ein radikalisch polymerisierbares, säuregruppenhaltiges Monomer. Säuregruppenhaltige Monomere werden im Folgenden auch als acide Monomere bezeichnet. Bevorzugte Säuregruppen sind Carbonsäuregruppen, Phosphonsäuregruppen, Phosphatgruppen und/oder Sulfonsäuregruppen, wobei diese Gruppen in der Säureform oder in Form eines Esters vorliegen können. Besonders bevorzugt sind Monomere mit Phosphonsäuregruppen oder Phosphatgruppen. Die Monomere können eine oder mehrere acide Gruppen aufweisen, bevorzugte sind Verbindungen mit 1 bis 2 aciden Gruppen.

Bevorzugte polymerisationsfähige Carbonsäuren sind Maleinsäure, Acrylsäure, Methacrylsäure, 2-(Hydroxymethyl)acrylsäure, 4-(Meth)acryloyloxyethyltrimellitsäure und das entsprechende Anhydrid, 10-Methacryloyloxydecylmalonsäure, N-(2-Hydroxy-3-methacryloyloxypropyl)-N-phenylglycin und 4-Vinylbenzoesäure.

Bevorzugte Phosphonsäuremonomere sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentyl-phosphonsäure 2-[4-(Dihydroxyphosphoryl)-2-oxa-butyl]-acrylsäure und 2-[2-Dihydroxyphosphoryl)-ethoxymethyl]-acrylsäure-2,4,6-trimethylphenylester.

Bevorzugte acide polymerisationsfähige Phosphorsäureester sind 2-Methacryloyloxypropylmono- und -dihydrogenphosphat, 2-Methacryloyloxyethylmono- und -dihydrogenphosphat, 2-Methacryloyloxyethyl-phenyl-hydrogenphosphat, Dipentaerythritolpentamethacryloyloxyphosphat, 10-Methacryloyloxydecyl-dihydrogenphosphat, Dipentaerythritolpentamethacryloyloxyphosphat, Phosphorsäuremono-(1-acryloyl-piperidin-4-yl)-ester, 6-(Methacrylamido)hexyldihydrogenphosphat und 1,3-Bis-(N-acryloyl-N-propyl-amino)-propan-2-yl-dihydrogenphosphat.

Bevorzugte polymerisationsfähige Sulfonsäuren sind Vinylsulfonsäure, 4-Vinylphenylsulfonsäure oder 3-(Methacrylamido)-propylsulfonsäure.

Zur Initiierung der radikalischen Photopolymerisation werden vorzugsweise Acylphosphinoxide, Bisacylphosphinoxide, Benzophenon, Benzoin sowie deren Derivate oder α-Diketone oder deren Derivate wie 9,10-Phenanthrenchinon, 1-Phenyl-propan-1,2-dion, Diacetyl oder 4,4-Dichlorbenzil eingesetzt. Bevorzugt werden Campherchinon und 2,2-Methoxy-2-phenyl-acetophenon und besonders bevorzugt α-Diketone in Kombination mit Aminen als Reduktionsmittel, wie z.B. 4-(Dimethylamino)-benzoesäureester, N,N-Dimethylaminoethylmethacrylat, N,N-Dimethyl-sym.-xylidin oder Triethanolamin, verwendet. Auch finden Kombinationen aus unterschiedlichen Photoinitiatoren Anwendung.

Als Initiatoren für eine sogenannte chemische Härtung werden Redox-Initiatorkombinationen, wie z.B. Kombinationen von Benzoylperoxid mit N,N-Dimethylsym.-xylidin oder N,N-Dimethyl-p-toluidin, verwendet. Darüber hinaus sind auch Redoxsysteme bestehend aus Peroxiden und Reduktionsmittel, wie z.B. Ascorbinsäure, Barbiturate oder Sulfinsäuren, besonders geeignet.

Weiterhin können die Dentalwerkstoffe zur Verbesserung der mechanischen Eigenschaften oder zur Einstellung der Viskosität organische oder anorganische partikuläre Füllstoffe enthalten. Bevorzugte anorganische partikuläre Füllstoffe sind amorphe kugelförmige Materialien auf der Basis von Oxiden, wie ZrO₂ und TiO₂, nanopartikuläre oder mikrofeine Füllstoffe, wie pyrogene Kieselsäure nanopartikuläres Al₂O₃, Ta₂O₅, Yb₂O₃, ZrO₂, Ag oder TiO₂ oder Mischoxide aus SiO₂, ZrO₂ und/oder TiO₂. oder Fällungskieselsäure sowie Minifüllstoffe, wie Quarz-, Glaskeramik- oder Glaspulver mit einer durchschnittlichen Teilchengrösse von 0,01 bis 1 µm sowie röntgenopake Füllstoffe, wie Ytterbiumtrifluorid oder nanopartikuläres Bariumsulfat. Besonders geeignet sind Füllstoffe, die mit polymerisationsfähigen Gruppen oberflächenmodifiziert sind.

Außerdem können die erfindungsgemässen Dentalmaterialien ein oder mehrere weitere Additive enthalten, die vorzugsweise aus Stabilisatoren, Inhibitoren, Aromastoffen, Farbstoffen, Pigmenten, fluoridionenabgebenden Additiven, optische Aufhellern, Weichmachern und/oder UV-Absorbern ausgewählt sind. Ein bevorzugter UV-Absorber ist 2-Hydroxy-4-methoxybenzophenon, bevorzugte Stabilisatoren sind 2,6-Di-tert-butyl-4-cresol und 4-Methoxyphenol.

Die Dentalwerkstoffe eignen sich insbesondere als Füllungsmaterialien und besonders als Beschichtungsmaterialien, Adhäsive, selbsthaftende und/oder selbstkonditionierende Befestigungszemente.

Die erfindungsgemäßen Dentalmaterialien enthalten vorzugsweise:
(a) 0,05 bis 50,0 Gew.-%, bevorzugt 0,5 bis 25,0 Gew.-% und besonders bevorzugt 2,0 bis 10,0 Gew.-% antimikrobielles, radikalisch polymerisierbares Makromer der Formel (I);
(b) 5 bis 95 Gew.-%, bevorzugt 5 bis 85 Gew.-% und besonders bevorzugt 5 bis 70 Gew.-% weiteres radikalisch polymerisierbares Monomer;
(c) 0,01 - 5,0 Gew.-% Initiator für die radikalische Polymerisation.

Außerdem enthalten die erfindungsgemäßen Dentalwerkstoffe vorzugsweise auch:
(d) 0 bis 60 Gew.-%, bevorzugt 5 bis 50 Gew.-% und besonders bevorzugt 5 bis 45 Gew.-% acides radikalisch polymerisierbares Monomer und/oder
(e) 0 bis 85 Gew.-% Füllstoff und/oder
(f) 0 bis 80 Gew.-%, bevorzugt 0 bis 60 Gew.-% und besonders bevorzugt 0 bis 40 Gew.-% Lösungsmittel und/oder
(g) 0,01 bis 5,0 Gew.-%, vorzugsweise 0,01 bis 3,0 Gew.-% weitere Additive.

Lösungsmittel werden hauptsächlich in Adhäsiven und Beschichtungsmaterialien eingesetzt. Bevorzugte Lösungsmittel sind Wasser, Methanol, Ethanol, Isopropanol, Ethylacetat, Aceton und Mischungen daraus.

Der genaue Füllstoffgehalt der erfindungsgemäßen Dentalwerkstoffe richtet sich nach der beabsichtigten Verwendung der Materialien. Dentalwerkstoffe zur Verwendung als Adhäsiv oder Beschichtungsmaterial enthalten vorzugsweise 0 bis 30 Gew.-% und Dentalmaterialien zur Verwendung als Zement oder Füllungsmaterial vorzugsweise 20 bis 85 Gew.-% Füllstoff.

Die vorliegende Erfindung betrifft auch die Verwendung von antimikrobiellen, radikalisch polymerisierbaren Makromeren der Formel (I) zur Herstellung von Dentalmaterialien, vorzugsweise den oben beschriebenen Dentalmaterialien.

Außerdem betrifft die Erfindung ein Verfahren zur Herstellung von Formkörpern, wie Kronen, Brücken, Inlays und künstlichen Zähnen, bei dem man einen erfindungsgemäßen Dentalwerkstoff auf an sich bekannte Weise zu dem Formkörper formt und dann zumindest teilweise, vorzugsweise vollständig härtet. Die Härtung erfolgt vorzugsweise durch radikalische Polymerisation.

Die Erfindung wird im Folgenden anhand von Beispielen näher erläutert.

### Ausführungsbeispiele

### Beispiel 1: Herstellung von Makromeren mit Polyoxazolinspacer beginnend mit Initiator

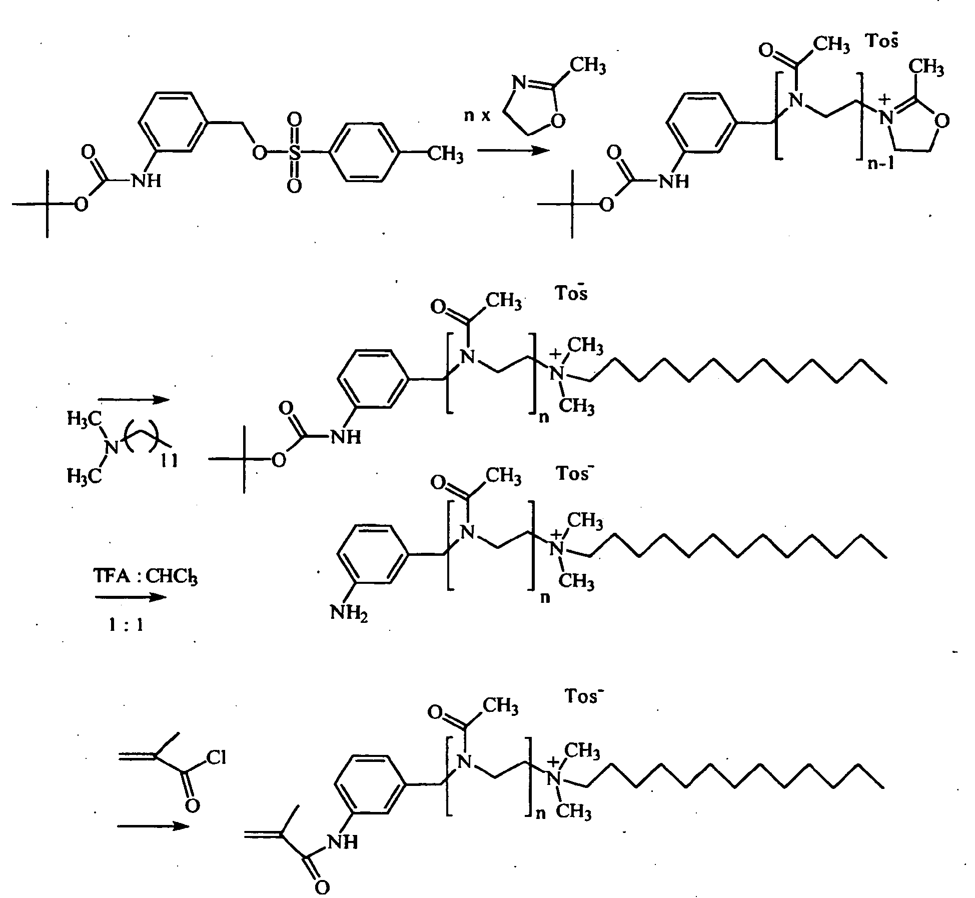

Zu einer Lösung aus 2-Methyl-1,3-oxazolin (5,0 g) in 25 ml CHCl₃ wurden bei 0 °C 0,2 g des Initiators 3-[(tert-Butoxycarbonyl)amino]benzyl-p-toluolsulfonat zugegeben. Nach 30 min wurde die Mischung auf 70 °C erhitzt, und man ließ den Ansatz für drei Tage rühren. Anschließend wurden 4,0 g (5,0 ml; 18,0 mmol) des Terminierungsreagenz *N,N-*Dimethyldodecylamin zugesetzt und für weitere 24 h bei 70 °C gerührt. Nach dreimaligen Umfällen aus Diethylether und Dialyse in Wasser bei RT erhielt man das leicht gelbliche, pulverförmige Polymer.

Abspaltung der Schutzgruppe: Ca. 3 g Polymer wurden in einer 1:1-Mischung aus Trifluoressigsäure und Dichlormethan (6 + 6 ml) gelöst und 30 min bei Raumtemperatur gerührt, wobei eine starke Gasentwicklung zu beobachten war. Anschließend wurde das auf diese Weise entschützte Polymer wie bei der eigentlichen Polymersynthese aufgearbeitet (Umkristallisation aus Diethylether) .

Umsetzung der freien Aminofunktion mit Methacrylsäurechlorid: Zu einer Lösung aus 2, 8 g des entschützten Polymers und 1,2 g Pyridin in 15 ml Chloroform wurden bei 0 °C 1,1 g Methacrylsäurechlorid zugetropft. Nach 2 h ließ man die Mischung auf RT kommen und rührte die Lösung für weitere 24 h. Nach dreimaligem Umfällen aus Diethylether und Dialyse in Wasser bei RT erhielt man 2,4 g (83 % d. Th.) des leicht gelblichen, pulverförmigen Makromers 1a.

Die Macromere 1b und 1c wurden auf die gleiche Weise hergestellt wie Macromer 1a, mit dem Unterschied, daß zur Herstellung von Macromer 1b ein Monomer/Initiator-Verhältnis (2-Methyl-1,3-oxazolin zu 3-[(tert-Butoxycarbonyl)amino]benzyl-p-toluolsulfonat) von 50:1 und im Fall von Macromer 1c ein Monomer/Initiator-Verhältnis von 100:1 gewählt wurde.

Die Charakterisierung der Makromere 1a, 1b und 1c erfolgte über FT-IR-, ¹H- und ¹³C-NMR-Untersuchungen sowie durch GPC-Messungen. Die Spacerlänge bzw. der Polymerisationsgrad ergibt sich aus dem Verhältnis Flächenintegrale der ¹H-NMR-Signale der Endgruppe zu denen der Spacersignale.
Makromer 1 a Mn = 2720 (g/mol)
   Polymerisationsgrad n = 25
Makromer 1 b Mn = 6120 (g/mol)
   Polymerisationsgrad n = 65
Makromer 1 c Mn = 11820 (g/mol)
   Polymerisationsgrad n = 132

### Beispiel 2: Herstellung von Makromeren mit Polyoxazolinspacer beginnend mit antimikrobieller Gruppe

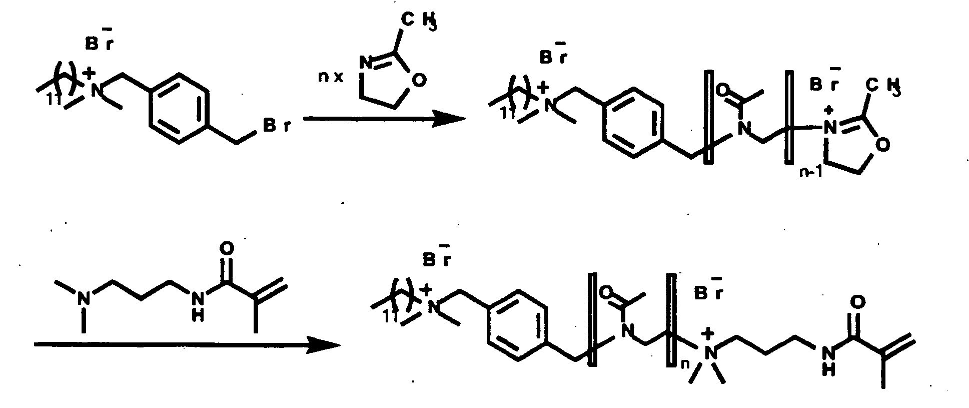

Zu einer Lösung aus 2-Methyl-1,3-oxazolin (5,0 g) in 25 ml CHCl₃ wurden bei 0 °C 0,3 g (1/100 Äquiv.) des Initiators (4-Brommethyl-benzyl)-*N,*N-dimethyldodecylammoniumbromid gegeben. Nach 30 min wurde die Mischung auf 70 °C erhitzt, und man ließ den Ansatz für drei Tage rühren. Anschließend wurden 3,1 g des Terminierungsreagenz *N,N*-Dimethylaminopropylmethacrylamid zugesetzt und für weitere 24 h bei 70 °C gerührt. Nach dreimaligem Umfällen aus Diethylether und Dialyse in Wasser bei RT erhielt man 4,7 g (87% d. Th.) des leicht gelblichen, pulverförmigen Makromers 2a.

Macromer 2b und 2c wurden auf die gleiche Weise hergestellt wie Macromer 2a, mit dem Unterschied, daß zur Herstellung von Macromer 2b ein Monomer/Initiator-Verhältnis (2-Methyl-1,3-oxazolin zu (4-Brommethyl-benzyl)-N,N-dimethyldodecylammoniumbromid) von 50:1 und im Fall von Macromer 2c ein Monomer/Initiator-Verhältnis von 100:1 gewählt wurde.

Die Charakterisierung der Makromere 2a, 2b und 2c erfolgte über FT-IR-, ¹H- und ¹³C-NMR-Untersuchungen sowie durch GPC-Messungen. Die Spacerlänge bzw. der Polymerisationsgrad ergibt sich aus dem Verhältnis Flächenintegrale der ¹H-NMR-Signale der Endgruppe zu denen der Spacersignale.
Makromer 2 a Mn = 2350 (g/mol)
   Polymerisationsgrad n = 20
Makromer 2 b Mn = 5170 (g/mol)
   Polymerisationsgrad n = 57
Makromer 2 c Mn = 10600 (g/mol)
   Polymerisationsgrad n = 117

### Beispiel 3: Bestimmung des MIC-Wertes der Makromere 1a - 1c und 2a - 2c

Die Bestimmung der Minimalen Inhibierungskonzentration (MIC-Wert; Minimal Inhibitory Concentration) erfolgte analog der in der Literatur beschriebenen Methode (A.H. Hogt, J. Dankert, J. Feijen, J. Biomed. Mater. Res. 1986, 20, 533).

50 ml steriles Standard-Kulturmedium (Firma Merck) wurden mit 100 µl einer Suspension von *Staphylokokkus aureus*-Zellen (ca. 10¹¹ Zellen pro ml) in PBS (Phosphat Buffer Saline), pH 7,0, angeimpft, für 6 h bei 37°C unter Schütteln inkubiert und danach auf 10⁵ Zellen pro ml mit Kulturmedium verdünnt (Konzentrationsbestimmung durch Extinktionsmessung bei 540 nm; E = 1 entspricht 10⁹ Zellen pro ml). In 1 ml der bakteriellen Suspension wurden dann 100 µl des zu testenden Makromers gelöst in Kulturmedium zugegeben. Die Mischung wurde erneut für 4-6 h unter Schütteln bei 37°C inkubiert, und bei deutlich sichtbarer Trübung der Kontrollprobe (bakterielle Suspension ohne Makromer) wurde die Extinktion bei 540 nm vermessen. Der MIC-Wert ist die minimale Makromerkonzentration, bei der der Extinktionswert der Probe mehr als 100 mal kleiner ist als der Wert der Kontrollprobe, d.h. bei der 99% der Bakterien am Wachstum gehindert werden. Die Ergebnisse sind in Tabelle 1 gezeigt.

**Tabelle 1**

| **MIC-Werte der Makromer 1a - 1c und. 2a - 2c** | | | |
|---|---|---|---|
| **Makromer MIC [mg/ml]** | | **MIC [µmol/l]** | |
| **1a** | 0 , | 2 | 85 |
| **1b** | 0 , | 4 | 69 |
| **1c** | 1 , | 0 | 84 |
| **2a** | 0 , 0 | 2 | 8 |
| **2b** | 0 , 0 | 4 | 8 |
| **2c** | 0 , 0 | 8 | 7 |

### Beispiel 4: Herstellung von dentalen Adhäsiven auf der Basis der Makromere 1a-c und 2a-c

Für die Untersuchung der antimikrobiellen Wirkung der Makromere der Formel (I) in Dentalwerkstoffen wurden Ahäsive mit den folgenden Zusammensetzungen hergestellt:
Adhäsiv A:
   (i) 14 Gew.-% des Vernetzers Glycerindimethacrylat (GDMA);
   (ii) 75 Gew.-% des hydrophilen Monomers 2-Hydroxyethylmethacrylat;
   (iii)10 Gew.-% des antimikrobiellen Makromers 1a, 1b oder 1c;
   (iv) 0,5 Gew.-% des Photoinitiators Genocure EPD und 0,5 Gew.-% des Initiators Campherchinon.
Adhäsiv B:
   (i) 15 Gew.-% des Vernetzers Glycerindimethacrylat (GDMA);
   (ii) 81 Gew.-% des hydrophilen Monomers 2-Hydroxyethylmethacrylat;
   (iii)3 Gew.-% des antimikrobiellen Makromers 2a, 2b oder 2c;
   (iv)0,5 Gew.-% des Photoinitiators Genocure EPD und 0,5 Gew.-% des Initiators Campherchinon.
Adhäsiv C:
   (i) 45 Gew.-% des Vernetzers Glycerindimethacrylat (GDMA);
   (ii) 24 Gew.-% des hydrophilen Monomers 2-Hydroxyethylmethacrylat;
   (iii) 30 Gew.-% des antimikrobiellen Makromonomers 2a, 2b oder 2c,
   (iv) 0,5 Gew.-% des Photoinitiators Genocure EPD und 0,5 Gew.-% des Initiators Campherchinon.

Die obigen Ahäsive wurden auf einen Objektträger gebracht und mit Hilfe einer UV-Lampe photopolymerisiert. Der entstandene klare Polymerfilm wurden anschließend zwei Tage mit Wasser gewaschen.

### Antimikrobielle Untersuchung

Jeweils ein modifizierter Objekträger wurde in einer Petrischale mit 30 ml einer *Staphylokokkus aureus* Suspension in PBS (-> 10⁷ Bakterienzellen/ml) bedeckt und anschließend unter leichtem Schütteln (50 rpm) für 1 h bei 37°C inkubiert. Dann wurde die Bakteriensuspension abgezogen, und der Objekträger wurde mit reinem PBS (15 + 15 ml) gewaschen. Nun wurde der Objekträger getrocknet, in eine neue Petrischale überführt, mit 30 ml 1,5%igen Nähragar bedeckt und anschließend bei 37°C inkubiert. Analog wurde jeweils eine Blindprobe getestet, bei der ein Objekträger verwendet wurde, der mit Adhäsiv ohne biozides Makromer beschichtet war. Nach 12 h zeigte sich auf der Blindprobe deutliches bakterielles Wachstum, wohingegen die Proben mit zugesetztem antibakteriellen Makromer in allen Fällen frei von jeglichem bakteriellen Befall blieben.

### Beispiel 5: Bestimmung der Hafteigenschaften und Lagerstabilität von dentalen Adhäsiven auf der Basis der Makromeren 2a-c

Zu Untersuchung der Hafteigenschaften auf Zahnschmelz und Dentin sowie der Lagerstabilität von Dentalwerkstoffe auf der Basis von Makromeren der Formel (I) wurde ein Adhäsiv der folgenden Zusammensetzung hergestellt:
Adhäsiv D:
   (i) 10 Gew.-% des Makromonomers 2a, 2b oder 2c;
   (ii) 10 Gew.-% des sauren Monomeren 2-[4-(Dihydroxyphosphoryl)-2-oxa-butyl]-acrylsäureethylester (DHPAE);
   (iii) 10 Gew.-% Hydroxyethylmethacrylat (HEMA) ;
   (iv) 30 Gew.% Bisphenol A-diglycidyletherdimethacrylat (Bis-GMA) ;
   (v) 20 Gew.% Glycerindimethacrylat (GDMA);
   (vi) 19 Gew.-% Ethanol;
   (vii) 0,5 Gew.-% des Photoinitiators Genocure und 0,5 Gew.-% des Initiators Campherchinon.

Es handelt sich um eine Zusammensetzung, die als dentales Adhäsiv für Schmelz und Dentin geeignet ist.

### Haftwertuntersuchungen (Scherhaftfestigkeit)

Für die Dentin bzw Schmelz-Haftmessungen wurden in Harz (Bühler Castolite-Resin) gegossene Rinderzähne verwendet. Zuerst wurde der eingegossene Rinderzahn approximal mit P500er Schleifpapier bis zum Dentin bzw. Schmelz abgeschliffen. Nach kurzem Feinschliff mit P1000er Schleifpapier und gründlichem Abwaschen unter fließendem Wasser, wurde der Zahn für die Prüfkörperherstellung präpariert.

Hierzu wurde wie folgt vorgegangen: Auf die Dentin- bzw. Schmelzoberfläche wurde Phosphorsäure-Ätzgel (Emailpräparator GS, Ivoclar Vivadent AG) aufgetragen und nach einer Einwirkzeit von 30s mit Wasser abgewaschen. Die so konditionierte Oberfläche wurde mit einem Papiertuch leicht trocken getupft und anschließend die zu untersuchende Adhäsivformulierung aufgetragen. Nach einer Einwirkzeit von 10 Sekunden wurde das Adhäsiv mit einer Polymerisationslampe (Astralis^{®} 7) für 20 Sekunden polymerisiert. Der so präparierte Zahn wurde in einer Scherhaftform gespannt und das Komposit mittels einer runden Delrinform von 4 mm Durchmesser in zwei Schichten (insgesamt max. 3 mm) aufgetragen, die jeweils 40s lang mit Astralis^{®} 7 (>500mW/cm²) belichtet wurden. Der so hergestellte Prüfkörper wurde entformt und sofort in Wasser gelegt. Nach Lagerung aller Prüfkörper (Anzahl der Prüfkörper n = 6) bei 37°C und 24h wurde die Scherhaftfestigkeit mittels einer Universalprüfmaschine (Zwick Z010) und einer Prüfkörpervorrichtung (Guillotine) bei einer Geschwindigkeit von 0,8 mm/Min. ermittelt (ISO/TS 11405; Dental materials - testing of adhesion to tooth structure. 2003).

Die Haftwerte der Adhäsive auf der Basis der Makromere 2a, 2b und 2c im Vergleich mit einer analogen Adhäsivformulierung ohne Zusatz von Makromonomer (Vergleichsprobe) sind in Tabelle 2 gezeigt.

**Tabelle 2**

| **Haftwerte von Adhäsiven auf der Basis der Makromere 2a-c** | | | |
|---|---|---|---|
| **M a k r o m e r** | **Mn** **(g/mol)** | **Schmelzhaftung** **(MPa)** | **Dentinhaftung** **(MPa)** |
| 2a | 2350 | 22.5 ± 4.2 | 15.9 ± 2.0 |
| 2b | 5170 | 20.6 ± 4.0 | 16.7 ± 1.6 |
| 2c | 10600 | 18.6 ± 4.3 | 18.9 ± 6.8 |
| Vergleichsprobe | - | 24.4 ± 4.8 | 19.6 ± 2.7 |

Wie aus der Tabelle 2 zu entnehmen ist, wirkt sich der Zusatz eines Makromeren der Formel 2a, 2b oder 2c nicht negativ auf die Dentin- bzw. Schmelzhafteigenschaften eines dentalen Adhäsivs aus.

### Beispiel 6: Herstellung eines dentalen Befestigungszements auf der Basis des Makromers 2c

Zu Untersuchung der mechanischen Eigenschaften von Dentalwerkstoffen auf der Basis von Makromeren der Formel (I) wurde ein dentaler Befestigungszement auf Kompositbasis mit der folgenden Zusammensetzung hergestellt:
(i) 1 Gew.-% des antibakteriellen Makromonomers 2c
(ii) 24 Gew.-% einer Mischung mehrfach funktioneller Monomere (Bis-GMA, UDMA und ethoxyliertes Bisphenol-A-dimethacrylat)
(iii) 6 Gew.-% einer Mischung monofunktioneller Methacrylate (2-Dimethylaminoethyl- und 2-Hydroxyethylmethacrylat)
(iv) 0,6 Gew.% Initiatoren (Bezoylperoxid und 3,5-Di-tert-butyl-N,N,-diethylanilin)
(v) 0, 1 Gew.% Stabilisatoren (2,2,6,6-Tetramethylpiperidin-1-oxyl und D-T-Butyl-P-Kresol)
(vi) 68,3 Gew.% anorganische Füllstoffe (Glaspulver, Ytterbiumtrifluorid, Si/Zr-Mischoxid, Titandioxid, pyrogene Kieselsäure)

Von diesem Befestigungszement und einer Vergleichsprobe wurde die Druckfestigkeit wie in EN ISO 9917-1:2003 im Anhang D beschrieben bestimmt. Die Ergebnisse sind in Tabelle 3 gezeigt.

**Tabelle 3**

| **Druckfestigkeit eines Zements auf der Basis des Makromers 2c** | | |
|---|---|---|
| **Z e m e n t** | **Mn (g/mol)** | **Druckfestigkeit** |
| mit Makromer 2c | 10.600 | 275 ± 30 MPa |
| Vergleichsprobe¹⁾ | - | 295 ± 25 MPa |

| | | |
|---|---|---|
| ¹⁾ Befestigungszement ohne antimikrobielles Makromer, das Makromer 2c wurde durch eine 1:1 Mischung von ethoxyliertem Bisphenol-A-dimethacrylat und 2 -Hydroxyethylmethacrylat ersetzt | | |

Wie aus der Tabelle 3 zu entnehmen ist, wirkt sich der Zusatz des Makromers 2c nicht signifikant auf die Druckfestigkeit und beeinträchtigt damit nicht die Eignung des Materials als Befestigungszement.

### Beispiel 7: Herstellung eines dentalen Adhäsivs auf der Basis des antimikrobiellen Monomers 12-Methacryloyloxydodecylpyridiuniumbromid (MDPB) (Vergleichsbeispiel)

Zu Vergleichszwecken wurde eine dentales Adhäsiv auf der Basis des antimikrobiellen Monomers 12-Methacryloyloxydodecylpyridiniumbromid (MDPB, synthetisiert nach EP 0 602 254 und durch ¹H-NMR-Spektroskopie charakterisiert) mit folgender Zusammensetzung hergestellt:
(i) 20 Gew.-% des Vernetzers Glycerindimethacrylat (GDMA);
(ii) 74 Gew.-% des hydrophilen Monomers 2-Hydroxyethylmethacrylat;
(iii) 5 Gew.-% des Monomers MDPB;
(iv) 0,5 Gew.-% des Photoinitiators Genocure EPD und 0,5 Gew.-% des Initiators Campherchinon.

Das Vergleichsmaterial zeigt eine ähnliche Zusammensetzung wie die in Beispiel 4 beschriebenen Adhäsive. Die Mischung wurde auf einen Objektträger gebracht und mit Hilfe einer UV-Lampe photopolymerisiert. Der entstandene klare Polymerfilm wurden anschließend zwei Tage mit Wasser gewaschen. Danach erfolgte die antimikrobielle Testung wie in Beispiel 4 beschrieben. Als Vergleich diente ein Objektträger, der mit Adhäsiv ohne das biozide Monomer MDPH beschichtet war. Die Vergleichsprobe zeigte deutliches bakterielles Wachstum, während die Probe mit MDPH keinen bakteriellen Befall aufwies.

Auffallend war, daß bei den Filmen mit MDPB-Zusatz nicht nur auf dem Film selbst kein bakterieller Befall zu sehen war, sondern daß sich um den Objektträger herum ein deutlich erkennbarer Hemmhof gebildet hatte. Ein Hemmhof ist ein eindeutiges Indiz dafür, daß ein Großteil des antimikrobiellen Wirkstoffes nicht immobilisiert wurde und aus dem Film in das Kulturmedium diffundiert. Eine solche Freisetzung von antimikrobiellen, potentiell toxischen Monomeren ist bei Dentalmaterialien unerwünscht.

## Patentansprüche

1. Dentalwerkstoff, der mindestens eine Verbindung der Formel (I) enthält,
[PG]ₘ-R¹-Z-SP-Y-R²- [WG]ₚ (I)
in der
m = 1, 2 oder 3;
p = 1, 2 oder 3;
R¹ = entfällt, ein linearer oder verzweigter C₁ bis C₂₀-Alkylenrest, der ein- oder mehrfach durch O, S, NH, SiR'₂, CONH, CONR', COO und/oder OCONH unterbrochen sein kann, ein substituierte oder unsubstituierter, aromatischer C₆ bis C₁₄-Rest oder eine Kombination davon;
R² = entfällt, ein linearer oder verzweigter C₁ bis C₂₀-Alkylenrest, der ein- oder mehrfach durch O, S, NH, SiR'₂, CONH, CONR', COO und/oder OCONH unterbrochen sein kann, ein substituierter oder unsubstituierter, aromatischer C₆ bis C₁₄-Rest oder eine Kombination davon;
PG = eine radikalisch polymerisierbare Gruppe;
SP = ein polymerer Spacer, der aus Polyethylenglycol-, Polypropylenglycol-, Polyglycerin-, Polyalkyloxazolinen-, Polyethyleniminen-, Polyacrylsäure-, Polymethacrylsäure-, Polyvinylalkohol-, Polyvinylacetat-, Poly-(2-hydroxyethyl)acrylat-, Poly-(2-hydroxyethyl)methacrylatgruppen, hydrophilen Polypeptidgruppen und Copolymeren der entsprechenden Monomere ausgewählt ist;
WG = eine antimikrobiell wirksame Gruppe;
Z = entfällt, O, S, eine Ester-, Amid- oder Urethangruppe;
Y = entfällt, O, S, eine Ester-, Amid- oder Urethangruppe;
R' = jeweils unabhängig ein linearer oder verzweigter C₁ bis C₂₀-Alkylrest, ein substituierter oder unsubstituierter Phenyl- oder Benzylrest;
und die ein Molekulargewicht von mindestens 1.000 hat.

2. Dentalwerkstoff nach Anspruch 1, bei dem die polymerisierbare Gruppe PG eine Vinyl-, Allyl-, Vinylether-, Styryl-, (Meth)acryloyl-, (Meth)acrylamid-, Vinylcyclopropylgruppe und/oder eine Gruppe der Formel (II) ist, 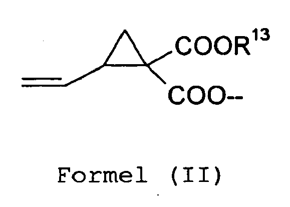 in der R¹³ Methyl oder Phenyl ist.

3. Dentalwerkstoff nach einem der vorhergehenden Ansprüche, bei dem die antimikrobiell wirksame Gruppe WG eine primäre, sekundäre, oder tertiäre Aminogruppe, eine kationische primäre, sekundäre, tertiäre oder quartäre Ammoniumgruppe, Phosphoniumgruppe oder Sulfoniumgruppe, eine Biguanidingruppe, ein antimikrobielles Peptid, ein Phenolrest oder Polyphenolrest und/oder ein Antibiotikum ist.

4. Dentalwerkstoff nach einem der vorhergehenden Ansprüche, bei dem der Spacer SP eine -[R³]_{q}- Gruppe ist, in der R³ -[CH₂-CH₂-O]-, - [CH (CH₃) -CH₂-O]-, -[CH(OH)CH₂] -, - [CH (COOH) CH₂]-, - [CH(COOCH₃)CH₂]-, - [C(CH₃) (COOH)CH₂]-, - [C(CH₃)(COOCH₃)CH₂] -, -[CH(COOCH₂CH₂OH)CH₂]-, -[C(CH₃)(COOCH₂CH₂OH)CH₂]-, -[CH(OCOCH₃)CH₂]-, -[CH₂-CH(OH)-CH₂-O]-, -[R⁴-N(-CO-R⁶)-R⁵]- oder eine Kombination daraus ist, wobei
R⁴ - entfällt, ein linearer oder verzweigter C₁ bis C₂₀-Alkylenrest, der durch ein oder mehrere O-Atome unterbrochen sein kann, ein substituierter oder unsubstituierter, aromatischer C₆ bis C₁₄-Rest,-(CH₂) 1₋4- oder entfällt;
R⁵ = entfällt, ein linearer oder verzweigter C₁ bis C₂₀-Alkylenrest, der durch ein oder mehrere O-Atome unterbrochen sein kann, ein substituierter oder unsubstituierter, aromatischer C₆ bis C₁₄-Rest,-(CH₂)₁₋₄- oder entfällt;
R⁶= ein linearer oder verzweigter C₁ bis C₂₀-Alkylrest, ein substituierter oder unsubstituierter, aromatischer C₆ bis C₁₄-Rest, C₁-C₃-Alkyl;
q = 10 bis 15.000.

5. Dentalwerkstoff nach einem der vorhergehenden Ansprüche, bei dem mindestens eine der Variablen eine der folgenden Bedeutungen,hat:
m = 1 oder 2;
p = 1 oder 2;
R¹ = entfällt, ein linearer oder verzweigter C₁ bis C₄-Alkylenrest, der einfach durch COO unterbrochen sein kann, Phenylen, Benzylen, -CH₂-Ph-CH₂-;
R² = entfällt, ein linearer oder verzweigter C₁ bis C₄-Alkylenrest, der einfach durch COO unterbrochen sein kann, Phenylen, Benzylen, -CH₂-Ph-CH₂-;
PG = CH₂=CR¹⁰-CO-X- mit R¹⁰ = H oder CH₃ und X = O, NH, NR¹², wobei R¹² ein linearer C₁ bis C₅-Alkylrest ist, CH₂=CH- oder Vinylcyclopropyl;
Z = entfällt, O, S, COO;
Y = entfällt, O, S, COO;
A⁻ = Cl⁻, Br⁻, I⁻, Mesylat (Mes), Tosylat (Tos) oder Triflat (Trf);
WG = -N⁺R⁷R⁸R⁹ A⁻, mit
R⁷ = H, ein linearer oder verzweigter C₁ bis C₂₀-Alkylrest, oder R⁷ bildet zusammen mit R⁸ und dem Stickstoffatom an den es gebunden ist ein aromatisches oder heteroaromatisches Ringsystem oder einen aromatischen oder nicht aromatischen Ring, vorzugsweise 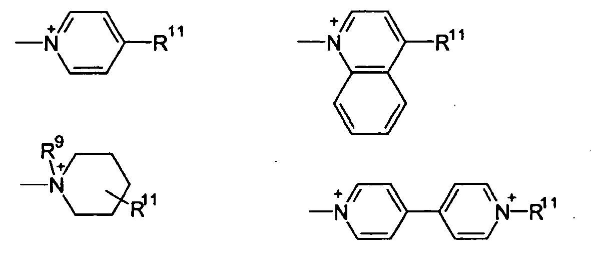 wobei R¹¹ ein linearer oder verzweigter C₁ bis C₂₀-Alkylrest ist;
R⁸ = H, ein linearer oder verzweigter C₁ bis C₂₀-Alkylrest oder R⁸ bildet zusammen mit R⁷ und dem Stickstoffatom an den es gebunden ist ein aromatisches oder heteroaromatisches Ringsystem oder einen aromatischen oder nicht aromatischen Ring;
R⁹ = entfällt, H, ein linearer oder verzweigter C₁ bis C₃₁-Alkylrest oder -(CH₂)ᵣ-H;
r = 5 bis 30;
-P⁺R^{7'}R^{8'}R^{9'} A⁻, mit
R^{7'} = H, ein linearer oder verzweigter C₁ bis C₂₀-Alkylrest;
R^{8'} = H, ein linearer oder verzweigter C₁ bis C₂₀-Alkylrest;
R^{9'} = entfällt, H, ein linearer oder verzweigter C₁ bis C₂₀-Alkylrest oder -(CH₂)ᵣ-H;
r = 5 bis 30; oder 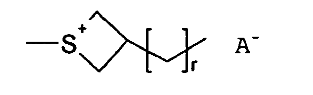
r = 5 bis 30.

6. Dentalwerkstoff nach Anspruch 5, bei dem die Verbindung der Formel (I) eine Verbindung der Formel (I") ist:

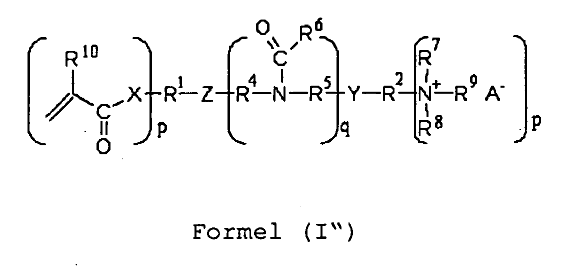

7. Dentalwerkstoff nach einem der vorhergehenden Ansprüche, bei dem die Verbindung der Formel (I) ein Molekulargewicht von mindestens 2.500 hat.

8. Dentalwerkstoff nach einem der vorhergehenden Ansprüche, bei dem die Verbindung der Formel (I) ein Molekulargewicht von mindestens 5.000 hat.

9. Dentalwerkstoff nach einem der vorhergehenden Ansprüche, der neben der Verbindung der Formel (I) mindestens ein weiteres radikalisch polymerisierbares Monomer enthält.

10. Dentalwerkstoff nach Anspruch 9, der mindestens ein weiteres radikalisch polymerisierbares Monomer mit 2 bis 3 radikalisch polymerisierbaren Gruppen enthält.

11. Dentalwerkstoff nach Anspruch 9 oder 10, der mindestens ein acides radikalisch polymerisierbares Monomer enthält.

12. Dentalwerkstoff nach einem der vorhergehenden Ansprüche, der mindestens einen Initiator für die radikalische Polymerisation enthält.

13. Dentalwerkstoff nach einem der vorhergehenden Ansprüche, der mindestens einen Füllstoff enthält.

14. Dentalwerkstoff nach einem der vorhergehenden Ansprüche, der
(a) 0,05 bis 50,0 Gew.-% radikalisch polymerisierbare Verbindung der Formel (I);
(b) 5 bis 95 Gew.-% weiteres radikalisch polymerisierbares Monomer; und
(c) 0,01 - 5,0 Gew.-% Initiator für die radikalische Polymerisation enthält.

15. Dentalwerkstoff nach Anspruch 14, der
(d) 0 bis 60 Gew.-%, acides radikalisch polymerisierbares Monomer enthält.

16. Dentalwerkstoff nach Anspruch 14 oder 15, der
(e) 0 bis 85 Gew.-% Füllstoff enthält.

17. Dentalwerkstoff nach einem der Ansprüche 14 bis 16, der
(f) 0 bis 80 Gew.-% Lösungsmittel enthält.

18. Verwendung einer Verbindung mit der Formel (I) zur Herstellung eines Dentalwerkstoffs.

19. Verwendung nach Anspruch 18 zur Herstellung eines Adhäsivs, Zements, Beschichtungsmaterials oder Füllungsmaterials.

20. Verfahren zur Herstellung eines Formkörpers bei dem man einen Dentalwerkstoff gemäß einem der Ansprüche 1 bis 17 zu einem Formkörper formt und anschließend härtet.

## Claims

1. Dental material comprising at least one compound of Formula (I),
[PG]ₘ-R¹-Z-SP-Y-R²-[WG]ₚ (I)
in which
m= 1,2 or 3;
p= 1, 2 or 3;
R¹ = is absent, a linear or branched C₁ to C₂₀ alkylene group that can be interrupted one or more times by O, S, NH, SiR'₂, CONH, CONR', COO and/or OCONH, a substituted or unsubstituted, aromatic C₆ to C₁₄ group or a combination thereof;
R² = is absent, a linear or branched C₁ to C₂₀ alkylene group that can be interrupted one or more times by O, S, NH, SiR'₂, CONH, CONR', COO and/or OCONH, a substituted or unsubstituted, aromatic C₆ to C₁₄ group or a combination thereof;
PG = a radically polymerisable group;
SP = a polymeric spacer selected from polyethylene glycol, polypropylene glycol, polyglycerol, polyalkyloxazoline, polyethylene imine, polyacrylic acid, polymethacrylic acid, polyvinyl alcohol, polyvinyl acetate, poly-(2-hydroxyethyl) acrylate, poly-(2-hydroxyethyl) methacrylate groups, polypeptide groups and copolymers of the corresponding monomers;
WG = an antimicrobially active group;
Z = is absent, O, S, an ester, amide or urethane group;
Y = is absent, O, S, an ester, amide or urethane group;
R' = independently of each other, a linear or branched C₁ to C₂₀ alkyl group, a substituted or unsubstituted phenyl or benzyl group;
and which has a molecular weight of at least 1000.

2. Dental material according to claim 1, in which the polymerisable group PG is a vinyl, allyl, vinyl ether, styryl, (meth)acryloyl, (meth)acrylamide, vinylcyclopropyl group and/or a group of Formula (II), 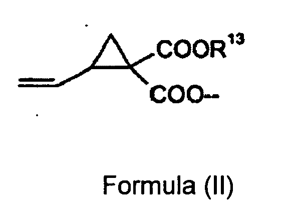 in which R¹³ is methyl or phenyl.

3. Dental material according to one of the previous claims, in which the antimicrobially active group WG is a primary, secondary, or tertiary amino group, a cationic primary, secondary, tertiary or quaternary ammonium, phosphonium or sulfonium group, a biguanidine group, an antimicrobial peptide, a phenol or polyphenol group and/or an antibiotic.

4. Dental material according to one of the previous claims, in which the spacer SP is an -[R³]q- group, in which R³ is -[CH₂-CH₂-O]-, -[CH (CH₃)-CH₂-O]-, -[CH(OH)CH₂]-, -[CH (COOH) CH₂]-, -[CH(COOCH₃)CH₂]-, - [C(CH₃) (COOH)CH₂]-, -[C(CH₃)(COOCH₃)CH₂]-, - [CH(COOCH₂CH₂OH)CH₂]-, -[C(CH₃)(COOCH₂CH₂OH)CH₂]-, - [CH(OCOCH₃)CH₂]-, -[CH₂-CH(OH)-CH₂-O]-, -R⁴-N(-CO-R⁶)-R⁵)- or a combination thereof, wherein
R⁴ = is absent, a linear or branched C₁ to C₂₀ alkylene group that can be interrupted by one or more O atoms, or is a substituted or unsubstituted, aromatic C₆ to C₁₄ group, -(CH₂)₁₋₄- or is absent;
R⁵ = is absent, a linear or branched C₁ to C₂₀ alkylene group that can be interrupted by one or more O atoms, or is a substituted or unsubstituted, aromatic C₆ to C₁₄ group, -(CH₂)₁₋₄- or is absent;
R⁶ = a linear or branched C₁ to C₂₀ alkyl group, a substituted or unsubstituted, aromatic C₆ to C₁₄ group, C₁-C₃ alkyl;
q = 10 to 15 000.

5. Dental material according to one of the previous claims, in which at least one of the variables has one of the following meanings:
m = 1 or 2;
p = 1 or 2;
R¹ = is absent, a linear or branched C₁ to C₄ alkylene group that can be interrupted once by COO, or is phenylene, benzylene, -CH₂-Ph-CH₂-;
R² = is absent, a linear or branched C₁ to C₄ alkylene group that can be interrupted once by COO, or is phenylene, benzylene, -CH₂-Ph-CH₂-;
PG = CH₂=CR¹⁰-CO-X- with R¹⁰ = H or CH₃ and X = O, NH, NR¹², wherein R¹² is a linear C₁ to C₅ alkyl group, CH₂=CH- or vinylcyclopropyl;
Z = is absent, O, S, COO;
Y = is absent, O, S, COO;
A- = Cl⁻, Br⁻, I⁻, mesylate (Mes), tosylate (Tos) or triflate (Trf);
WG = -N⁺R⁷R⁸R⁹ A⁻, with
R⁷ = H, a linear or branched C₁ to C₂₀ alkyl group, or R⁷ forms together with R⁸ and the nitrogen atom to which it is bonded an aromatic or heteroaromatic ring system or an aromatic or non-aromatic ring, preferably 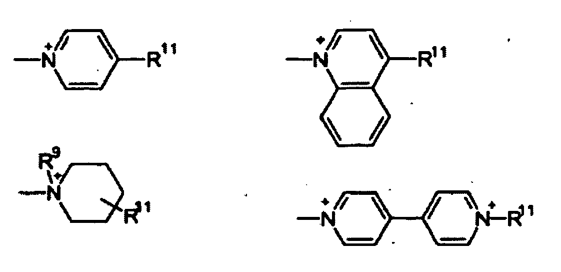 wherein R¹¹ is a linear or branched C₁ to C₂₀ alkyl group;
R⁸ = H, a linear or branched C₁ to C₂₀ alkyl group or R⁸ forms together with R⁷ and the nitrogen atom to which it is bonded an aromatic or heteroaromatic ring system or an aromatic or non-aromatic ring;
R⁹ = is absent, H, a linear or branched C₁ to C₃₁ alkyl group or-(CH₂)r-H;
r = 5 to 30;
-P⁺R^{7'}R^{8'}R^{9'} A⁻, with
R^{7'} = H, a linear or branched C₁ to C₂₀ alkyl group;
R^{8'} = H, a linear or branched C₁ to C₂₀ alkyl group;
R^{9'} = is absent, H, a linear or branched C₁ to C₂₀ alkyl group or-(CH₂)ᵣ-H;
r = 5 to 30; or 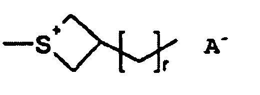
r = 5 to 30.

6. Dental material according to claim 5, in which the compound of Formula (I) is a compound of Formula (I"):

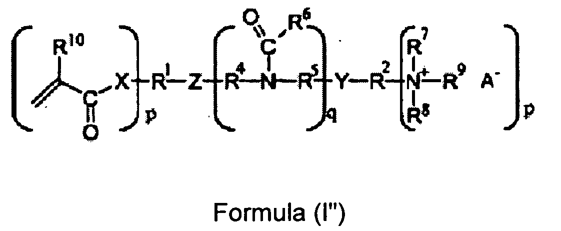

7. Dental material according to any one of the previous claims, in which the compound of Formula (I) has a molecular weight of at least 2 500.

8. Dental material according to one of the previous claims, in which the compound of Formula (I) has a molecular weight of at least 5 000.

9. Dental material according to one of the previous claims comprising, in addition to the compound of Formula (I), at least one further radically polymerisable monomer.

10. Dental material according to claim 9 comprising at least one further radically polymerisable monomer containing 2 to 3 radically polymerisable groups.

11. Dental material according to claim 9 or 10 comprising at least one acidic radically polymerisable monomer.

12. Dental material according to one of the previous claims comprising at least one initiator for the radical polymerisation.

13. Dental material according to one of the previous claims comprising at least one filler.

14. Dental material according to one of the previous claims comprising
(a) 0.05 to 50.0 wt.% of a radically polymerisable compound of Formula (I);
(b) 5 to 95 wt.-% of a further radically polymerisable monomer; and
(c) 0.01 - 5.0 wt.% of an initiator for the radical polymerisation.

15. Dental material according to claim 14 comprising
(d) 0 to 60 wt.% of an acidic, radically polymerisable monomer.

16. Dental material according to claim 14 or 15 comprising
(e) 0 to 85 wt.% filler.

17. Dental material according to one of claims 14 to 16 comprising
(f) 0 to 80 wt.% solvent.

18. Use of a compound with Formula (I) for the preparation of a dental material.

19. Use according to claim 18 for the preparation of an adhesive, cement, coating material or filling material.

20. Process for the preparation of a moulding in which a dental material according to one of claims 1 to 17 is shaped to form a moulding and then cured.

## Revendications

1. Matériau dentaire, qui comprend au moins un composé de formule (I) :
[PG]ₘ-R¹-Z-SP-Y-R²-[WG]ₚ (I)
dans laquelle :
- l'indice m vaut 1, 2 ou 3 ;
- l'indice p vaut 1, 2 ou 3 ;
- R¹ ne représente rien ou représente un groupe alcanediyle en C₁₋₂₀ linéaire ou ramifié, dont la chaîne peut être interrompue par un ou plusieurs chaînons ou fragments symbolisés par -O-, -S-, -NH-, - Si(R')₂-, -CONH-, -CONR'-, -COO- et/ou -OCONH-, ou représente un groupe aromatique en C₆₋₁₄, doté ou non de substituant(s), ou encore une combinaison de tels groupes ;
- R² ne représente rien ou représente un groupe alcanediyle en C₁₋₂₀ linéaire ou ramifié, dont la chaîne peut être interrompue par un ou plusieurs chaînons ou fragments symbolisés par -O-, -S-, -NH-, - Si(R')₂-, -CONH-, -CONR'-, -COO- et/ou -OCONH-, ou représente un groupe aromatique en C₆₋₁₄, doté ou non de substituant(s), ou encore une combinaison de tels groupes ;
- PG représente un groupe qui peut donner lieu à une polymérisation radicalaire ;
- SP représente un écarteur polymère, choisi parmi des fragments de type polyéthylèneglycol, polypropylèneglycol, polyglycérol, poly(alkyl-oxazoline), poly(éthylène-imine), poly(acide acrylique), poly(acide méthacrylique), poly(alcool vinylique), poly(acétate de vinyle), poly-(acrylate de 2-hydroxy-éthyle) ou poly(méthacrylate de 2-hydroxy-éthyle), les fragments polypeptidiques hydrophiles et les fragments de types copolymères des monomères correspondants ;
- WG représente un fragment doté d'une activité anti-microbienne ;
- Z ne représente rien ou représente un chaînon symbolisé par -O- ou - S- ou un groupe de type ester, amide ou uréthane ;
- Y ne représente rien ou représente un chaînon symbolisé par -O- ou - S- ou un groupe de type ester, amide ou uréthane ;
- et R' représente, indépendamment en chaque occurrence, un groupe alkyle en C₁₋₂₀ linéaire ou ramifié, ou un groupe phényle ou benzyle, doté ou non de substituant(s) ;
lequel composé présente une masse molaire d'au moins 1000.

2. Matériau dentaire conforme à la revendication 1, dans lequel le groupe polymérisable représenté par PG est un groupe vinyle, allyle, vinyl-oxy, styryle, acryloyle, méthacryloyle, acrylamido, méthacrylamido et/ou vinyl-cyclopropyle et/ou un groupe de formule (II) : 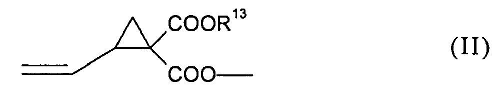 dans laquelle R¹³ représente un groupe méthyle ou phényle.

3. Matériau dentaire conforme à l'une des revendications précédentes, dans lequel le fragment doté d'une activité anti-microbienne, représenté par WG, est un groupe de type amine primaire, secondaire ou tertiaire, un groupe cationique de type ammonium, phosphonium ou sulfonium, primaire, secondaire, tertiaire ou quaternaire, un groupe de type bis(guanidine), un peptide anti-microbien, un reste de phénol ou de poly-phénol et/ou un antibiotique.

4. Matériau dentaire conforme à l'une des revendications précédentes, dans lequel l'écarteur représenté par SP est un groupe symbolisé par -[R³]_{q}- où R³ représente :
- un groupe de formule -[CH₂-CH₂-O]-, -[CH(CH₃)-CH₂-O]-, - [CH(OH)-CH₂]-, -[CH(COOH)-CH₂]-, -[CH(COOCH₃)-CH₂]-, - [C(CH₃)(COOH)-CH₂]-, -[C(CH₃)(COOCH₃)-CH₂]-, - [CH(COOCH₂CH₂OH)-CH₂]-, -[C(CH₃)(COOCH₂CH₂OH)-CH₂]-, - (CH(OCOCH₃)-CH₂)- ou -[CH₂-CH(OH)-CH₂-O]-,
- un groupe symbolisé par -[R⁴-N(-CO-R⁶)-R⁵-]-,
- ou une combinaison de tels groupes ;
étant entendu que :
R⁴ ne représente rien ou représente un groupe alcanediyle en C₁₋₂₀ linéaire ou ramifié, dont la chaîne peut être interrompue par un ou plusieurs atomes d'oxygène, ou un groupe aromatique en C₆₋₁₄, doté ou non de substituant(s), ou représente un groupe de formule -(CH₂)₁₋₄ ou ne représente rien ;
R⁵ ne représente rien ou représente un groupe alcanediyle en C₁₋₂₀ linéaire ou ramifié, dont la chaîne peut être interrompue par un ou plusieurs atomes d'oxygène, ou un groupe aromatique en C₆₋₁₄, doté ou non de substituant(s), ou représente un groupe de formule -(-CH₂)₁₋₄ ou ne représente rien ;
et R⁶ représente un groupe alkyle en C₁₋₂₀ linéaire ou ramifié ou un groupe aromatique en C₆₋₁₄, doté ou non de substituant(s), ou représente un groupe alkyle en C₁₋₃ ;
et l'indice q vaut de 10 à 15 000.

5. Matériau dentaire conforme à l'une des revendications précédentes, dans lequel au moins l'un des indices et symboles a l'une des valeurs et significations suivantes :
- l'indice m vaut 1 ou 2 ;
- l'indice p vaut 1 ou 2 ;
- R¹ ne représente rien ou représente un groupe alcanediyle en C₁₋₄ linéaire ou ramifié, dont la chaîne peut être interrompue par un groupe symbolisé par -COO-, ou représente un groupe phénylène, benzylène ou xylylène ;
- R² ne représente rien ou représente un groupe alcanediyle en C₁₋₄ linéaire ou ramifié, dont la chaîne peut être interrompue par un groupe symbolisé par -COO-, ou représente un groupe phénylène, benzylène ou xylylène ;
- PG représente un groupe vinyle ou vinyl-cyclopropyle, ou un groupe de formule -CH₂=CR¹⁰-CO-X- où R¹⁰ représente un atome d'hydrogène ou un groupe méthyle et X représente un atome d'oxygène ou un groupe symbolisé par NH ou NR¹² où R¹² représente un groupe alkyle en C₁₋₅ linéaire ;
- Z ne représente rien ou représente un chaînon symbolisé par -O- ou - S- ou un groupe symbolisé par -COO- ;
- Y ne représente rien ou représente un chaînon symbolisé par -O- ou - S- ou un groupe symbolisé par -COO- ;
- A⁻ représente un anion chlorure, bromure, iodure, méthanesulfonate (Mes), para-toluènesulfonate (Tos) ou trifluorométhanesulfonate (Trf) ;
- et WG représente :
soit un groupe de formule -N⁺R⁷R⁸R⁹ A⁻, dans laquelle
- R⁷ représente un atome d'hydrogène ou un groupe alkyle en C₁₋₂₀ linéaire ou ramifié, ou bien R⁷ et R⁸ représentent des entités qui constituent, conjointement avec l'atome d'azote auquel elles sont liées, un système cyclique aromatique ou hétéroaromatique ou un cycle aromatique ou non aromatique, et de préférence, un groupe de formule : 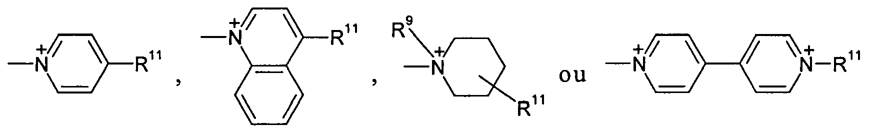
où R¹¹ représente un groupe alkyle en C₁₋₂₀ linéaire ou ramifié,
- R⁸ représente un atome d'hydrogène ou un groupe alkyle en C₁₋₂₀ linéaire ou ramifié, ou bien R⁸ et R⁷ représentent des entités qui constituent, conjointement avec l'atome d'azote auquel elles sont liées, un système cyclique aromatique ou hétéroaromatique ou un cycle aromatique ou non aromatique,
- et R⁹ ne représente rien ou représente un atome d'hydrogène ou un groupe alkyle en C₁₋₃₁ linéaire ou ramifié, ou représente un groupe de formule -(CH₂)ᵣ-H où l'indice r vaut de 5 à 30 ;
soit un groupe de formule -P⁺R ⁷'R⁸'R⁹' A⁻, dans laquelle
- R⁷' représente un atome d'hydrogène ou un groupe alkyle en C₁₋₂₀ linéaire ou ramifié,
- R⁸' représente un atome d'hydrogène ou un groupe alkyle en C₁₋₂₀ linéaire ou ramifié,
- et R⁹' ne représente rien ou représente un atome d'hydrogène ou un groupe alkyle en C₁₋₂₀ linéaire ou ramifié, ou représente un groupe de formule -(CH₂)ᵣ-H où l'indice r vaut de 5 à 30 ;
soit un groupe de formule 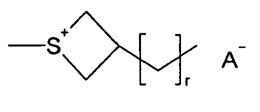 dans laquelle l'indice r vaut de 5 à 30.

6. Matériau dentaire conforme à la revendication 5, dans lequel le composé de formule (I) est un composé de formule (I") :

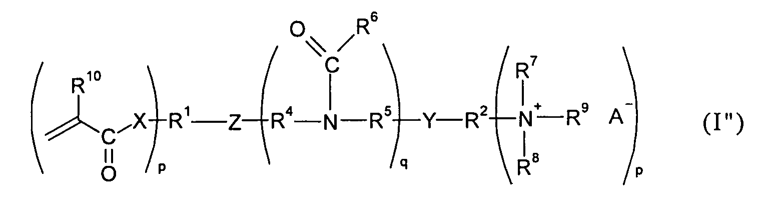

7. Matériau dentaire conforme à l'une des revendications précédentes, dans lequel le composé de formule (I) présente une masse molaire d'au moins 2500.

8. Matériau dentaire conforme à l'une des revendications précédentes, dans lequel le composé de formule (I) présente une masse molaire d'au moins 5000.

9. Matériau dentaire conforme à l'une des revendications précédentes, qui contient, en plus du composé de formule (I), au moins un autre monomère polymérisable par voie radicalaire.

10. Matériau dentaire conforme à la revendication 9, qui contient au moins un autre monomère polymérisable par voie radicalaire, comportant 2 ou 3 groupes pouvant donner lieu à une polymérisation radicalaire.

11. Matériau dentaire conforme à la revendication 9 ou 10, qui contient au moins un monomère acide polymérisable par voie radicalaire.

12. Matériau dentaire conforme à l'une des revendications précédentes, qui contient au moins un amorceur de polymérisation radicalaire.

13. Matériau dentaire conforme à l'une des revendications précédentes, qui contient au moins une charge.

14. Matériau dentaire conforme à l'une des revendications précédentes, qui contient :
a) de 0,05 à 50,0 % en poids d'un composé polymérisable par voie radicalaire, de formule (I) ;
b) de 5 à 95 % en poids d'un autre monomère polymérisable par voie radicalaire ;
c) et de 0,01 à 5,0 % en poids d'un amorceur de polymérisation radicalaire.

15. Matériau dentaire conforme à la revendication 14, qui contient :
d) de 0 à 60 % en poids d'un monomère acide polymérisable par voie radicalaire.

16. Matériau dentaire conforme à la revendication 14 ou 15, qui contient :
e) de 0 à 85 % en poids d'une charge.

17. Matériau dentaire conforme à l'une des revendications 14 à 16, qui contient :
f) de 0 à 80 % en poids d'un solvant.

18. Utilisation d'un composé de formule (I) pour la préparation d'un matériau dentaire.

19. Utilisation, conforme à la revendication 18, pour la préparation d'un adhésif, d'un ciment, d'un matériau de revêtement ou d'un matériau de remplissage.

20. Procédé de fabrication d'un corps façonné, dans lequel on façonne un matériau dentaire, conforme à l'une des revendications 1 à 17, pour en faire un corps façonné que l'on fait ensuite durcir.
